# EUROPEAN PATENT APPLICATION

(11) **EP 4 488 440 A1**
(43) Date of publication of application: **08.01.2025**
(21) Application number: 23781347.2
(22) Date of filing: 29.03.2023
(51) Int. Cl.: D06F 75/20, D06F 75/26, D06F 75/28, D06F 39/00, A61L 2/07

(54) **CLOTHING TREATMENT APPARATUS**

(30) Priority: 29.03.2022 KR 20220039194
(71) Applicant: LG Electronics Inc., Yeongdeungpo-gu Seoul 07336 (KR)
(72) Inventor: SONG, Sungho, Seoul 08592 (KR); LEE, Jaemyoung, Seoul 08592 (KR); SHIN, Woosup, Seoul 08592 (KR)
(74) Representative: Vossius & Partner Patentanwälte Rechtsanwälte mbB
(86) International application number: PCT/KR2023/004174
(87) International publication number: WO 2023/191492

(57) **Abstract**

The present invention relates to a clothing treatment apparatus comprising: a cabinet; a door rotatably coupled to the cabinet; an inner case provided in the cabinet to provide an accommodation space in which clothing can be placed; a refresh module comprising a base portion disposed lower than the inner case, a circulation duct which is attached to the base or extends from the base portion to provide a flow path through which the air inside the inner case is circulated, a heat supply unit which heats the air flowing along the circulation duct, and a steam supply unit which is attached in the circulation duct and provided to supply steam into the inner case; and an iron module comprising a steam iron which is disposed at the left or right side of the refresh module to supply the steam onto the surface of clothing, wherein the steam iron is provided to be withdrawn to the front portion of the iron module.

## Description

### [Technical Field]

The present disclosure relates to a clothing treatment apparatus and a method for controlling the same. More specifically, the present disclosure relates to a clothing treatment apparatus and a method for controlling the same that may perform a refreshing cycle such as sterilization, wrinkle removal, deodorization, and drying of laundry by supplying steam and hot air to the laundry.

### [Background]

Recently, a clothing treatment apparatus that may perform laundry management (refreshment) such as sterilization, deodorization, and drying of laundry via supply of hot air and steam without washing the laundry with water and detergent has appeared. Examples of such existing clothing treatment apparatuses include Korean Patent Application Publication Nos. 10-2020-0057545 and 10-2011-0067832.

The clothing treatment apparatus is able to manage the laundry without water and the detergent, so that the laundry is not damaged and a time it takes to manage the laundry and then wear the laundry again is shorter compared to laundry treatment of a washing machine and a dryer.

In addition, because the clothing treatment apparatus manages the laundry while the laundry is hung as it is without being folded, wrinkles are not formed in the laundry, but the wrinkles are able to be removed during the laundry management process.

However, although the clothing treatment apparatus is able to manage the laundry in a not wet state, the wrinkles may not be completely removed from the laundry. In addition, when removing the wrinkles from the laundry without refreshing the laundry, operating the clothing treatment apparatus is disadvantageous in terms of time and cost.

Therefore, even when the clothing treatment apparatus is equipped, it is common to additionally place an iron for each household to remove the wrinkles from the laundry by supplying heat and steam to a surface of the laundry.

When the iron is equipped as a steam iron that may spray steam, the wrinkles from the laundry are able to be removed by supplying heat and steam to the laundry while the laundry is hung on a clothes hanger or the like.

Although the iron and the clothing treatment apparatus have something in common in that they are able to manage the dry laundry, it was inefficient to purchase the steam iron and the clothing treatment apparatus together, and there is a problem of lowering space utilization because the steam iron and the clothing treatment apparatus should be disposed separately.

Recently, to solve such problem, a clothing treatment apparatus that is equipped with the steam iron to manage the laundry has appeared. See Korean Patent No. 10-1265602 and Korean Patent Application Publication No. 10-2022-0056090.

The clothing treatment apparatus is provided to perform a refreshing cycle by supplying hot air and steam as needed after hanging the laundry in an inner casing (inside) without folding the same, and also remove the wrinkles by supplying heat and steam to the surface of the laundry with the steam iron.

Such existing clothing treatment apparatus has effects that a user does not need to purchase the separate steam iron and a separate place to store the steam iron is not needed.

Such clothing treatment apparatus is able to be provided such that the steam iron is in communication with the inner casing. Therefore, the user is able to hang the laundry in the inner casing and then pull the steam iron out toward the inner casing to supply heat and steam to the surface of the laundry. In this case, there is an advantage of not having to have a separate space other than the inner casing to treat the laundry with the steam iron.

However, when the refreshing is performed, the inner casing is sealed from the outside, and hot air and steam are supplied into the inner casing. Therefore, when the steam iron is in communication with or exposed to the inner casing, the steam iron is corroded or damaged by hot air and steam supplied to the inner casing.

In particular, when foreign substances removed from the laundry remain inside the inner casing, the foreign substances are exposed to the steam iron and the steam iron is rather contaminated.

To solve such problem, a clothing treatment apparatus in which the steam iron is withdrawn via a side surface of a cabinet so as not to be in communication with the inner casing has appeared. See Chinese Patent Publication No. 112826234.

Such clothing treatment apparatus has an advantage that the steam iron is not exposed to air, steam, or the foreign substances inside the inner casing, so that there are no problems such as corrosion or contamination.

However, the clothing treatment apparatus requires a separate component to hang the laundry to use the steam iron.

In addition, there is a limitation that a separate home appliance is not able to be disposed or the clothing treatment apparatus is not able to be disposed near a wall to withdraw the steam iron or place a hanging member that supports the laundry via or at the side surface of the clothing treatment apparatus.

In addition, the existing clothing treatment apparatus has a fundamental problem that when the existing clothing treatment apparatus is not equipped with the steam iron, the clothing treatment apparatus equipped with the steam iron has to be repurchased.

### [Summary]

### [Technical Problem]

The present disclosure is to place an iron module together in a clothing treatment apparatus that may perform refreshing, such as sterilization, deodorization, and drying, of laundry.

The present disclosure is to provide a clothing treatment apparatus that may block an iron module from being exposed to hot air and steam supplied from a clothing treatment apparatus.

The present disclosure is to provide a clothing treatment apparatus that may block an iron module from being exposed to foreign substances removed during a refreshing cycle.

The present disclosure is to provide a clothing treatment apparatus that may additionally install only an iron module even in a model in which the iron module is not installed.

The present disclosure is to allow a home appliance or a wall to be disposed on each of both sides of a clothing treatment apparatus in which an iron module is installed.

### [Technical Solutions]

To solve the above-described problems, the present disclosure includes a steam iron that is stored in a clothing treatment apparatus, but is able to be withdrawn forward of the clothing treatment apparatus.

The steam iron is disposed under an inner casing in which laundry is hung, so as to be prevented from being exposed to hot air or steam supplied into the inner casing, and also be prevented from coming into contact with foreign substances discharged from the laundry.

The steam iron is provided so as to be withdrawn forward only when a door that closes the inner casing is opened, and is completely accommodated inside a machine room when the door is closed.

The door may seal the inner casing such that air inside the inner casing is not discharged outside the inner casing when the inner casing is closed.

As a result, even when the steam iron is disposed in the clothing treatment apparatus, a possibility of contamination or electrical leakage by fluid inside the inner casing may be completely blocked.

Specifically, the clothing treatment apparatus of the present disclosure includes a refreshing module including a base disposed below the inner casing, a circulation duct mounted or extended on the base to provide a flow channel where air inside the inner casing circulates, a heat supply portion that heats air flowing along the circulation duct, and a steam supply portion that is mounted on the circulation duct and supplies steam into the inner casing.

Further, the clothing treatment apparatus of the present disclosure includes an iron module including a steam iron that is disposed on a left side or a right side of the refreshing module and supplies steam to a surface of the laundry.

The steam iron may be withdrawn forward of the iron module.

The iron module may include a housing body disposed on a left side or a right side of the circulation duct to accommodate the steam iron therein, and an open surface defined in a front surface of the housing body and allowing the steam iron to be withdrawn forward of a machine room therethrough.

The open surface may be exposable to the outside when the door is opened.

The iron module may further include an withdrawal portion accommodated in the housing body to withdraw the steam iron, and a cover disposed in front of the withdrawal portion to close the open surface.

The withdrawal portion may be provided to expose the steam iron to the outside of the open surface by being withdrawn from the housing body via the open surface or rotatably coupled in front of the housing body.

The door may include a sealing member disposed on an inner surface thereof and in contact with a front surface of the inner casing.

The sealing member may include a partition member disposed between the inner casing and a machine room.

The door may further include a protruding surface protruding from the inner surface thereof and at least partially inserted into the inner casing.

The protruding surface may be disposed above the sealing member.

The protruding surface may protrude from the inner surface of the door such that at least a portion thereof faces a bottom surface of the inner casing when the door is closed.

A width of the protruding surface may be set to be equal to or smaller than a width of an opening of the inner casing.

The steam iron may be set to operate and the refreshing module may be set to be prevented from operating when the door is opened.

The steam iron may be set to stop operating or be blocked from operating and the refreshing module may be set to be operable when the door is closed.

The iron module may further include a heating module disposed on a left side or a right side of the circulation duct to generate steam to be supplied to the steam iron. The heating module may be disposed behind the steam iron.

The steam iron may include a cable extending from the heating module and allowing heat and steam to flow therethrough, and a handle coupled to a distal end of the cable and withdrawn from the housing forward of a machine room to supply heat and steam to the surface of the laundry.

To solve the above-described problems, the present disclosure provides a clothing treatment apparatus in which the steam iron is disposed to be exposable forward of the machine room.

The steam iron may be disposed to be exposable forward of the machine room when the door is opened.

The steam iron may be stored inside the machine room when the door is closed.

The machine room may include a circulation duct circulating air inside the inner casing, and a heat supply portion that heats air flowing along the circulation duct.

The iron module may further include a housing disposed outside the circulation duct and accommodating the steam iron therein, and the steam iron may be disposed to be exposable forward of the machine room from the housing.

An entirety of the housing may be positioned below a bottom surface of the inner casing.

To solve the above-described problems, the present disclosure provides a clothing treatment apparatus including a housing body disposed on a left side or a right side of the circulation duct to accommodate the steam iron therein, and an open surface defined in a front surface of the housing body and exposing the steam iron forward of a machine room.

The housing body may accommodate the steam iron therein so as to block the steam iron from being withdrawn via a top surface and both side surfaces thereof.

To solve the above-described problems, the present disclosure provides a clothing treatment apparatus further including a circulation duct circulating air inside the inner casing, a heat supply portion that heats air flowing along the circulation duct, a steam supply portion that supplies steam into the inner casing, a water supply tank disposed in front of the circulation duct to store water to be supplied to the steam supply therein, and a drainage tank collecting water condensed in the circulation duct and disposed on an opposite side of the water supply tank.

The water supply tank and the drainage tank may both be disposed in front of the circulation duct, and the iron module may be disposed on a left side or a right side of the circulation duct.

The water supply tank and the drainage tank may be disposed adjacent to each other, and the iron module may be disposed to be spaced apart from one of the water supply tank and the drainage tank.

The iron module may be disposed closer to the water supply tank than to the drainage tank.

The iron module may further include a heating module that receives water from the water supply tank and generates steam to be supplied to the steam iron. The heating module may be disposed closer to the water supply tank than to the drainage tank.

### [Advantageous Effects]

The present disclosure has the effect of placing the iron module together in the clothing treatment apparatus that may perform the refreshing, such as the sterilization, the deodorization, and the drying, of the laundry.

The present disclosure has the effect of blocking the iron module from being exposed to hot air and steam supplied from the clothing treatment apparatus.

The present disclosure has the effect of blocking the iron module from being exposed to the foreign substances removed during the refreshing cycle.

The present disclosure has the effect of additionally installing only the iron module even in the model in which the iron module is not installed.

The present disclosure has the effect of allowing the home appliance or the wall to be disposed on each of both sides of the clothing treatment apparatus in which the iron module is installed.

### [Brief Description of the Drawings]

FIG. 1 shows an outer appearance of a clothing treatment apparatus of the present disclosure.
FIG. 2 shows interior of a cabinet of a clothing treatment apparatus of the present disclosure.
FIG. 3 shows an iron module of a clothing treatment apparatus of the present disclosure.
FIG. 4 shows a front side of a machine room of a clothing treatment apparatus of the present disclosure.
FIG. 5 shows a rear side of the machine room.
FIG. 6 shows structures of a circulation duct and a base in a machine room of a clothing treatment apparatus of the present disclosure.
FIG. 7 shows a structure of a circulation duct of a clothing treatment apparatus of the present disclosure.
FIG. 8 shows a structure of a controller installation portion disposed on a base of a clothing treatment apparatus of the present disclosure.
FIG. 9 shows an outer appearance of a water supply and drainage system of a clothing treatment apparatus of the present disclosure.
FIG. 10 shows an installation structure of a steam supply that receives water from a water supply tank.
FIG. 11 shows an outer appearance of the steam supply.
FIG. 12 shows an internal structure of a steam supply.
FIG. 13 shows a structure of supplying water to the steam supply.
FIG. 14 shows a flow channel structure of the steam nozzle.
FIG. 15 shows a drainage structure of a clothing treatment apparatus of the present disclosure.
FIG. 16 shows a drainage tank installation structure of a clothing treatment apparatus of the present disclosure.
FIG. 17 shows in detail a structure of a backflow portion of a clothing treatment apparatus of the present disclosure.
FIG. 18 shows a lower configuration of a bottom surface of an inner casing.
FIG. 19 shows a structure of the ironing module in detail.
FIG. 20 shows a water supply and drainage structure of the heating module.
FIG. 21 shows a structure in which the water supply structure and the water supply are in communication with each other.
FIG. 22 shows a structure in which the drainage structure and the drainage may be in communication with each other.
FIG. 23 shows a structure in which the drainage structure and the drainage are in communication with each other.
FIG. 24 shows an embodiment in which the iron module I of the present disclosure is installed in a machine room.
FIG. 25 shows a structure of the steam iron.
FIG. 26 shows characteristics of a direction in which the steam iron is withdrawn.
FIG. 27 shows a structure in which the steam iron is protected from foreign substances.
FIG. 28 shows a structure and an arrangement of an iron module and a controller.
FIG. 29 is a diagram illustrating arrangement of the controllers.
FIG. 30 shows an embodiment in which the iron module is equipped in a drawer manner.
FIG. 31 shows an embodiment in which a steam iron is stored in the withdrawal portion.
FIG. 32 shows an embodiment in which a steam iron in FIG. 30 is stored and withdrawn.
FIG. 33 shows another embodiment of the withdrawal portion.
FIG. 34 shows a specific structure of FIG. 33.
FIG. 35 shows an embodiment in which a steam iron in FIG. 33 is stored and withdrawn.

### [Best Model

Hereinafter, embodiments disclosed herein will be described in detail with reference to the attached drawings. In the present document, identical or similar components are assigned identical or similar reference numerals even in different embodiments, and descriptions thereof are replaced with the first description. A singular expression used herein includes a plural expression unless the context clearly indicates otherwise. In addition, when describing the embodiments disclosed herein, when it is determined that a detailed description of a related known technology may obscure the gist of the embodiments disclosed herein, the detailed description thereof will be omitted. In addition, it should be noted that the attached drawings are only intended to facilitate easy understanding of the embodiments disclosed herein, and the technical ideas disclosed herein should not be construed as being limited by the attached drawings. In the present document, an X-axis means a front and rear direction of a clothing treatment apparatus, a Y-axis means a width direction of the clothing treatment apparatus, and a Z-axis means a height direction of the clothing treatment apparatus.

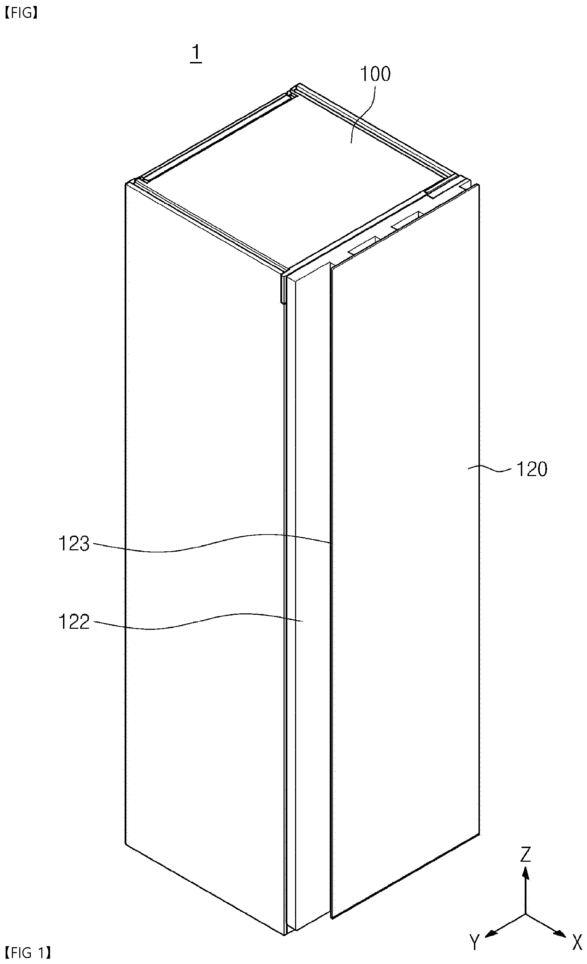

FIG. 1 shows an outer appearance of a clothing treatment apparatus of the present disclosure.

The clothing treatment apparatus of the present disclosure may include a cabinet 100 forming an outer appearance, and a door 120 rotatably coupled to the cabinet 100.

The door 120 may include a main body 121 forming a front surface of the cabinet 100, and an installation body 122 extending from one side of the main body 121 and where a display for displaying information of the clothing treatment apparatus may be installed.

The cabinet 100 may be provided such that a height is greater than a width in a left and right direction and a thickness in a front and rear direction. Accordingly, in the clothing treatment apparatus, even long laundry may be hung inside the cabinet 100 without being folded.

The installation body 122 may be extended from the main body 121 while forming a step rearward of the cabinet 100.

The installation body 122 may be made of a material or may have a color different from that of the main body 121. In addition, the installation body 122 may be made of a translucent material through which light emitted from the display may be transmitted.

A handle 123 may be disposed in an area where the installation body 122 and the main body 121 are stepped.

The handle 123 may be disposed on one surface of the main body 121 to extend forward of the installation body 122 in parallel with the main body 121. As a result, the handle 123 may be disposed to at least partly overlap the installation body 122 in the front and rear direction, and may define a space that the user may grip.

The cabinet 100 and the door 120 may be made of a metal material.

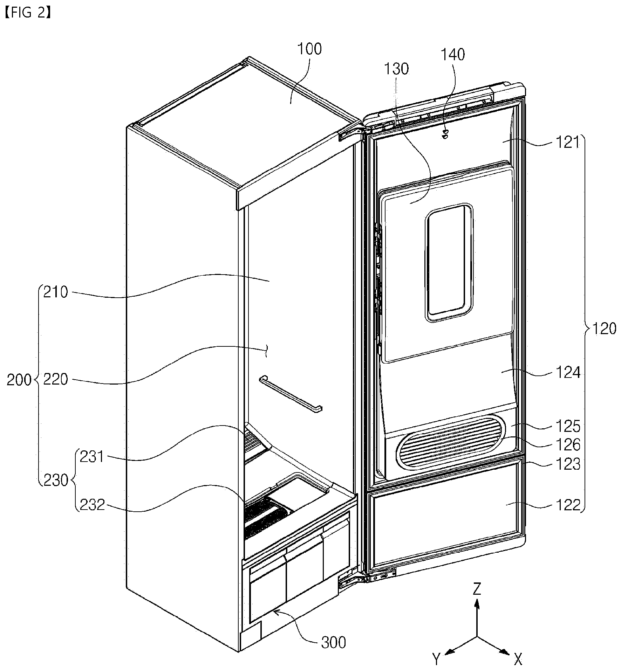

FIG. 2 shows interior of a cabinet of a clothing treatment apparatus of the present disclosure.

An inner casing 200 that has an accommodating space 220 for accommodating the laundry defined therein and defines an opening 210 in a front side through which the laundry is introduced may be disposed inside the cabinet 100.

The inner casing 200, which has a rectangular parallelepiped shape with an open front surface, may have a height smaller than that of the cabinet 100. As a result, an area in which a machine room 300 to be described below may be installed may be secured in the lower portion of the cabinet 100.

The inner casing 200 may have the height greater than a width and a thickness. As a result, the laundry may be hung inside the accommodating space 220 without being folded or wrinkled.

The inner casing 200 may be made of a plastic resin material, and may be made of a reinforced plastic resin material that does not deform even when exposed to air at a temperature higher than a room temperature or heated air (hereinafter, hot air) and steam or moisture.

A hanger where the laundry may be hung in the accommodating space 220 may be disposed in an upper portion of an inner surface of the inner casing 200. The hanger may be formed in a clothes hanger shape and may be fixed to a top surface of the inner casing 200. Because of the hanger, the laundry may be disposed in the unfolded state and in a state of being suspended in air within the accommodating space 220.

A plurality of the hangers may be disposed along a width direction of the inner casing 200. As a result, a plurality of laundry pieces may be hung, spaced apart from each other within the inner casing 200.

The hanger may be provided as a moving hanger that reciprocates or rotates in a reciprocating manner in the width direction within the inner casing 200. The clothing treatment apparatus of the present disclosure may shake the laundry inside the inner casing because of the hanger, and the laundry may be shaken inside the accommodating space 220, thereby removing foreign substances, dust, and the like, and also removing wrinkles formed on the laundry.

The clothing treatment apparatus of the present disclosure may be equipped with the machine room 300 in which various apparatuses are installed, which supply high-temperature air and heated air (hereinafter, hot air) to the accommodating space 220, supply steam to the accommodating space 220, or purify or dehumidify outside air of the cabinet 100.

The machine room 300 may be disposed separately or partitioned from the inner casing 200, but may be in communication with the accommodating space 220.

The machine room 300 may be disposed under the inner casing 200. Accordingly, when hot air and steam having a low specific gravity are supplied to the inner casing 200, hot air and steam may naturally rise in the accommodating space 220 and be supplied to the hung laundry.

The inner casing 200 may be partitioned and separated from the machine room 300 via a bottom surface 230. However, a plurality of through-holes may be defined in the bottom surface 230 to be in communication with the machine room 300.

To this end, the inner casing 200 may have a plurality of through-holes 230 that extend through one surface thereof and are in communication with the machine room 300.

Via the through-holes 230, air in the accommodating space 220 may be supplied to the machine room 300 and at least one of hot air and steam generated in the machine room 300 may be supplied to the accommodating space 220.

The through-holes 230 may include an inflow hole 232 extending through the bottom surface 230 of the inner casing 200 and allowing air inside the inner casing 200 to be discharged or sucked into the machine room 300, and an exhaust hole 231 extending through the bottom surface of the inner casing 200 and allowing hot air generated in the machine room 300 to be discharged.

The exhaust hole 231 may be defined to be biased rearward in the bottom surface of the inner casing 200. In addition, the inflow hole 232 may be defined to be biased forward in the bottom surface of the inner casing 200. As a result, a separation spacing between the inflow hole 232 and the exhaust hole 231 may be secured on the bottom surface 230 of the inner casing 200, and hot air supplied from the exhaust hole 231 may be prevented from being directly discharged to the inflow hole 232.

The through-holes 230 may further include a steam hole 233 into which steam generated in a steam supply portion 800 to be described below is supplied. The steam hole 233 may be defined closer to the exhaust hole 231 than to the inflow hole 232. For example, the steam hole 233 may be defined on one side of the exhaust hole 231. As a result, steam discharged from the steam hole 233 may be prevented from flowing directly into the inflow hole 232.

The door 120 may be rotatably coupled to the cabinet 100.

A height of the door 120 may correspond to the height of the cabinet 100. As a result, the door 120 may open and close the opening 210, and may also shield the machine room 300.

When the door 120 is closed, the opening 210 and the machine room 300 may be prevented from being exposed forward.

The door 120 may include a door body 121 forming a main body and a sealing member 123 coupled to an inner surface of the door body 121 to seal the opening 210.

The sealing member 123 may be disposed in an area facing a periphery of the opening 210 on the inner surface of the door body 121 to seal the opening 210.

In one example, the door body 121 may include a protective panel 122 that shields the machine room 300 and protects the machine room 300 under the sealing member 123 that seals the opening 210.

The sealing member 123 may extend below the opening 210 to also seal a front periphery of the machine room 300.

The door 120 may include a protruding portion 125 that protrudes from the inner surface of the door body 121 such that at least a portion thereof may be inserted into the opening 210.

The protruding portion 125 may protrude from the door body 121 to an extent that it is disposed in front of the inflow hole 232 when the door 120 closes the opening 210.

The protruding portion 125 may have a width corresponding to a width of the opening 210. The protruding portion 125 may be disposed closer to a lower portion of the opening 210 or the bottom surface 230 of the inner casing 200 than to an upper end of the door body 121.

As a result, hot air and steam in the accommodating space 220 of the inner casing may be guided to be introduced into the inflow hole 232, and may be prevented from being discharged out of the opening 210 and being exposed to the machine room 300.

A height of the protruding portion 125 may be smaller than 1/3 of the height of the inner casing 200, and a thickness of the protruding portion 125 protruding from the door body 121 may be smaller than a spacing from a front edge of the bottom surface 230 to the inflow hole 232.

The door body 121 may further include a curved surface 124 extending toward the protruding portion 125 on the inner surface thereof. The curved surface 124 may be formed in a downwardly convex shape. The curved surface 124 may induce hot air and steam supplied to the accommodating space 220 to circulate inside the accommodating space 220.

The clothing treatment apparatus of the present disclosure may include a pressurizer 130 that is rotatably coupled to the inner surface of the door body 121 to pressurize the laundry, and a fixer 140 that may hang the laundry at a vertical level higher than that of the pressurizer 130.

The pressurizer 130 may pivot in a width direction of the door 120 to pressurize the laundry hung on the fixer 140.

Accordingly, by pressing the laundry hung on the fixer 140 with the pressurizer 130, the wrinkles in the laundry may be removed, and intended creases may also be formed in the laundry.

The curved surface 124 may extend from a lower end of the pressurizer 130 to the protruding portion 125. Accordingly, when water condensed in the laundry hung on the fixer 140 flows along the pressurizer 130 or the inner surface of the door body 121, water may flow along the curved surface 124 and be guided to the bottom surface 230 and may be prevented from flowing to the opening 210 or the sealing member 123. As a result, the machine room 300 may be prevented from being contaminated with water, steam, hot air, the foreign substances, and the like.

The protruding portion 125 may have an exposed surface disposed parallel to a rear surface of the inner casing 200, and a bottom surface extending from a lower portion of the exposed surface may be disposed parallel to the bottom surface 230 of the inner casing.

The exposed surface of the protruding portion 125 may have a through-hole 126 that extends through the exposed surface to allow air to enter and exit, and may have a duct through which air may flow in a space defined by the protruding portion 125, the curved surface 124, and the door body 121.

The door body 121 may have a communication hole in communication with the duct in an area of the inner surface corresponding to the pressurizer 130.

Accordingly, hot air and steam flowing in the inner casing 200 may be introduced into the through-hole 126 and discharged via the communication hole to dry the laundry hung on the pressurizer 130 or circulate air inside the inner casing 200.

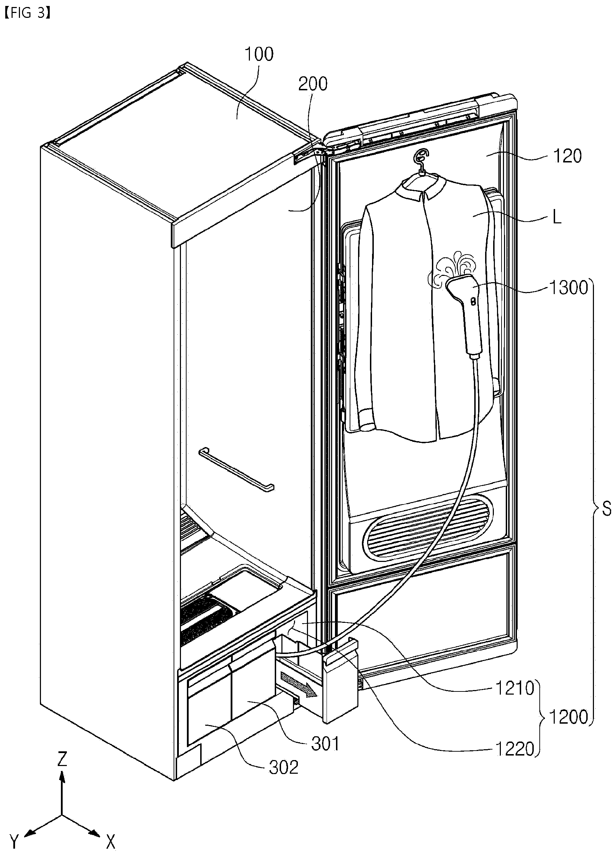

FIG. 3 shows an outer appearance of the machine room 300.

The machine room 300 supplies at least one of hot air and steam to the inner casing 200. To this end, the machine room 300 should be supplied with water necessary to generate steam and should collect steam supplied to the inner casing 200 or water condensed from the laundry.

To this end, the machine room 300 may include the water supply tank 301 that stores water to be used for steam, and the drainage tank 302 that collects water condensed inside the clothing treatment apparatus.

The water supply tank 301 and the drainage tank 302 may be disposed in a front portion of the machine room 300. As a result, it may be easy to fill the water supply tank 301 with water or empty water in the drainage tank 302, and the clothing treatment apparatus may be disposed without being limited by locations of a water source and a drain.

The clothing treatment apparatus of the present disclosure may further include an iron module S that supplies at least one of heat and steam generated in the machine room to the laundry. The iron module S may supply heat and steam to a surface of the laundry.

The iron module S may include a steam iron 1300 that may spray or supply at least one of heat and steam to the surface of the laundry, and a housing 1200 that stores the steam iron 1300 inside the machine room 300.

The housing 1200 may include a housing body 1210 that is mounted in the machine room 300 and provides a space in which the steam iron 1300 is stored, and an open surface 1220 that is defined in a front surface of the housing body 1210 and through which the steam iron 1300 is inserted and withdrawn.

The steam iron 1300 may be withdrawn from the machine room 300 while being withdrawn forward of the housing body 1210. The steam iron 1300 may reach the surface of the laundry hung on the fixer 140 or the hanger when withdrawn forward of the machine room 300.

The steam iron 1300 may transmit heat and steam to an upper end of the pressurizer 130 of the door 120, which is located above a midpoint in a height direction of the inner casing 200. The steam iron 1300 may be in contact with the surface of the laundry or may be disposed close to the laundry to directly spray heat and steam to the surface of the laundry.

For example, the steam iron 1300 may reach the hanger or the fixer 140. In addition, the steam iron 1300 may move to a top surface of the accommodating space 220 or an upper end of the inner surface of the door 120 when withdrawn from the housing 1200.

The steam iron 1300 may supply heat to the surface of the laundry or may spray steam to the surface of the laundry. The steam iron 1300 may be equipped as a general steam iron or a general steamer. The steam iron 1300 may be equipped as any component as long as it satisfies a condition of removing the wrinkles that have occurred in the laundry.

Laundry L fixed to the hanger or the fixer 140 may be supported on the inner surface of the door 120 or an inner surface of the inner casing 200 and may be exposed to at least one of heat and steam supplied from the steam iron 1300. In this process, the laundry L may be refreshed or wrinkles thereof may be removed.

The ironing module S may be exposed to the front surface of the machine room 300, because the steam iron 1300 should be withdrawn from the machine room 300 to the laundry hung in the inner casing 200 or on the door 120.

For example, a front surface of the ironing module S may be disposed on one side of the water supply tank 301 or the drainage tank 302, and both side surfaces thereof may be disposed inside the machine room 300.

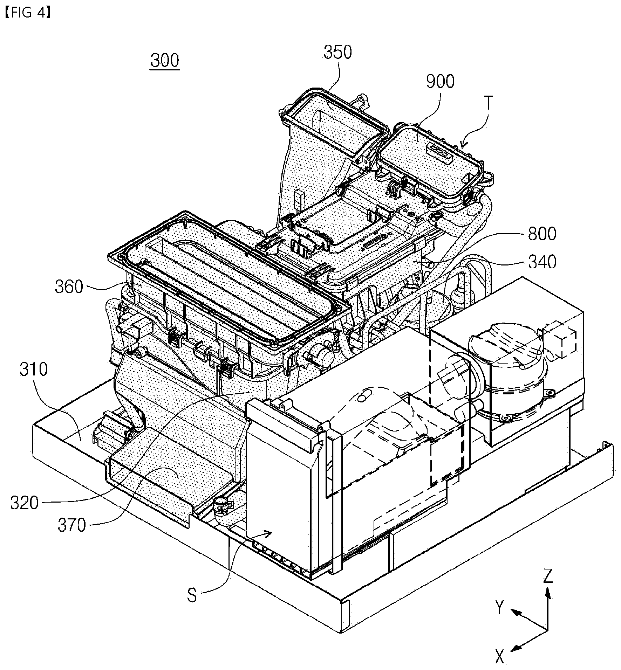

FIG. 4 shows a structure of a machine room of a clothing treatment apparatus of the present disclosure.

The machine room 300 may include therein a refreshing module T equipped as a component that may supply hot air and steam to the laundry hung in the accommodating space 220, circulate air inside the accommodating space, or circulate air outside the cabinet, and the ironing module S including the steam iron 1300.

The refreshing module T may include all apparatuses and components that may supply at least one of hot air and steam into the inner casing 200.

Specifically, the machine room 300 may include a base 310 on which a space where the various apparatuses described above may be supported or installed, a circulation duct 320 that is installed on or extended from the base 310 and provides a flow channel through which air inside the inner casing 200 or air outside the cabinet 100 flows, a blower 350 that is mounted on the circulation duct 320 and provides power to allow air to flow, a heat supply portion 340 that cools and heats air flowing along the circulation duct 320 to generate hot air, and the steam supply portion 800 that is supported on the base 310 or mounted on the circulation duct 320 and supplies steam into the inner casing 200.

The base 310 may be formed as a plate on which the various apparatuses are installed.

The circulation duct 320 may form a flow channel through which air introduced from the inner casing 200 or the outside of the cabinet 100 flows, and may be formed in a casing shape with an open top.

The heat supply portion 340 may include a heat exchanger disposed inside the circulation duct 320 to cool air, condense moisture, and reheat air, and a compressor disposed outside the circulation duct 320 to receive a refrigerant from the heat exchanger or supply the refrigerant.

The refreshing module may further include an outside air duct 370 that sucks outside air in front of the circulation duct 320 and guides sucked air into the circulation duct 320.

The circulation duct 320 may be in communication with the outside air duct 370 and selectively suck outside air.

The water supply tank and the drainage tank may also be included in the refreshing module T.

The water supply tank and the drainage tank may be detachably coupled to a front surface of the circulation duct 320. For example, the water supply tank 301 and the drainage tank 302 may be seated and disposed on the outdoor air duct 370.

The circulation duct 320 may be coupled to the base 310, but may be formed integrally with the base 310. For example, the base 310 and the circulation duct 320 may be simultaneously manufactured via injection molding.

The refreshing module T may include a base cover 360 disposed to be in communication with the circulation duct 320 and the inflow hole 232.

The base cover 360 may be coupled to an upper portion of the circulation duct 320 to guide air sucked in the inflow hole 232 into the circulation duct 320.

The base cover 360 may block air inside the circulation duct 320 from being discharged to the outside by shielding a top surface of the circulation duct 320. A lower portion of the base cover 360 and the top surface of the circulation duct 320 may form one surface of a flow channel of the circulation duct 320.

The base cover 360 may include an inlet 362 that allows the inflow hole 232 and the circulation duct 320 to be in communication with each other therein. The inlet 362 may be formed in a duct shape to serve as an intake duct that delivers air inside the inner casing 200 to the circulation duct 320.

The steam supply portion 800 may be connected to the water supply tank 301 and receive water to generate steam. The steam supply portion 800 may be seated and disposed on the circulation duct 320. The steam supply portion 800 may be disposed behind the base cover 360.

The refreshing module T may further include a steam nozzle 900 that receives steam from the steam supply portion 800 and supplies steam into the inner casing 200. The steam nozzle 900 may be disposed between the steam supply portion 800 and the steam hole 233.

Water that has not been discharged from the steam nozzle 900 to the steam hole 233 but condensed may be recovered again to the steam supply portion 800.

The blower 350 may allow the circulation duct 320 and the exhaust hole 231 to be in communication with each other.

The ironing module S may be disposed on a left or right side of the refreshing module T inside the machine room 300. Specifically, the ironing module S may be disposed outside the circulation duct 320. Thus, the ironing module S may not obstruct a flow of air through the circulation duct 320.

The ironing module S may be disposed at one side of the base 310 to be parallel thereto along a length direction of the circulation duct 320

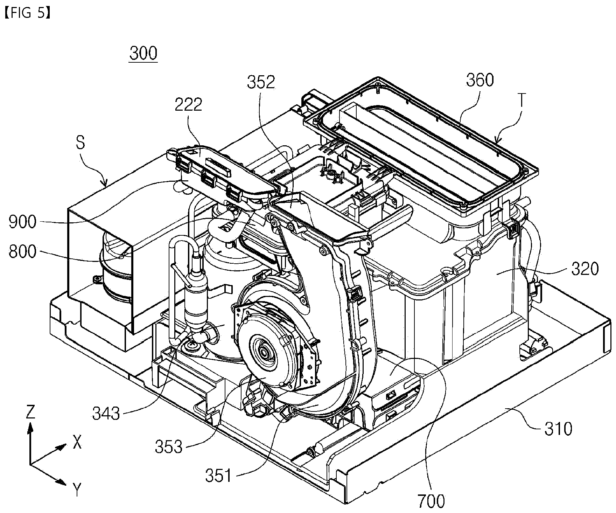

FIG. 5 is a rear view of the machine room.

The blower 350 may include a blower fan 353 that provides power for air inside the circulation duct 320 to flow in one direction, and a fan housing 351 that accommodates the blower fan 353 therein and is coupled to or extended from the circulation duct 320.

The blower 350 may include an exhaust duct 352 that allows the circulation duct 320 and the exhaust hole 231 to be in communication with each other.

The exhaust duct 352 may be formed with a cross-section extending from the fan housing 351 toward exhaust hole 231 in an area size corresponding to that of exhaust hole 231.

As a result, air inside the inner casing 200 may be introduced via the base cover 360, pass through the circulation duct 320, and then be supplied back into the inner casing 200 via a fan installation portion 350.

The heat supply portion 340 may include a compressor 343 installed on the base 310 to exchange heat with air flowing through the circulation duct 320. The compressor 343 may be disposed on a left or right side of the circulation duct 320, and may be disposed between the circulation duct 320 and the ironing module S.

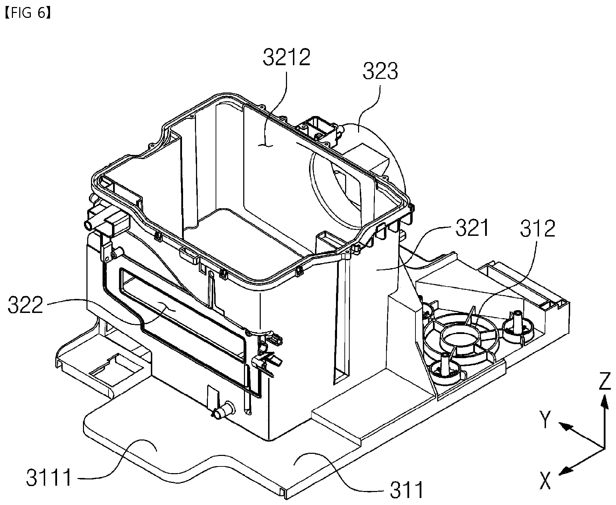

FIG. 6 shows structures of a circulation duct and a base in a machine room of a clothing treatment apparatus of the present disclosure.

The base 310 may form a bottom surface of the clothing treatment apparatus.

The base 310 may include a base bottom 311 forming a support surface. The base bottom 311 may form the bottom surface of the clothing treatment apparatus.

In one example, the base bottom 311 may be seated on a top surface of the bottom surface of the cabinet 100 separately disposed to form the bottom surface of the clothing treatment apparatus.

The base 310 may be integrally formed with the circulation duct 320 that forms at least a portion of the flow channel through which air flows. The circulation duct 320 may be formed by extending upward from the base bottom 311.

The circulation duct 320 may include a duct body 321 that extends from the base bottom 311 to form the flow channel, a heat exchanger installation portion 3212 that provides a space in which an evaporator 341 or a condenser 342 is installed inside the duct body 321, and an air discharger 323 that is disposed behind the duct body 321 and discharges air in the duct body 321 to the blower 350.

The air discharger 323 may be formed in a pipe shape that extends rearward from the duct body 321. A diameter of the air discharger 323 may be smaller than a width of the duct body 321.

The air discharger 323 may be connected to the blower 350. Air discharged from the air discharger 323 may be guided into the inner casing 200 via the blower 350.

The circulation duct 320 may further include an outside air intake portion 322 defined by extending through a front surface of the duct body 321. The outside air intake portion 322 may be in communication with the outside air duct 370. The outside air duct 370 may be seated and supported in front of the outside air intake portion 322.

The circulation duct 320 may be installed with a damper that opens and closes the outside air intake portion 322. Opening and closing of the damper may allow or block inflow of outside air into the circulation duct 320.

The base 310 may include a compressor installation portion 312 that provides a space in which the compressor 343 is installed. The compressor installation portion 312 may be formed at one side of the base bottom 311 and may be formed integrally with the base bottom 311.

The compressor installation portion 312 may also have a protrusion formed that may support the compressor 343. The compressor installation portion 312 may be disposed to be biased to a rear side of the base 310. The compressor installation portion 312 may be disposed to at least partially overlap the air discharger 323 in a width direction.

A buffer member that reduces vibration transmitted from the compressor 343 may be installed in the compressor installation portion 312. The buffer member may be fixed to the protrusion.

The base 310 may include a compressor installation portion 312 in which the compressor 343, which supplies the refrigerant to the heat exchanger 341 and 343, is installed. The compressor installation portion 312 may be disposed outside the circulation duct 320.

The ironing module S may be seated on the compressor installation portion 312 or at one side of the base 310. The ironing module S may also be seated on the base 310. For example, the base bottom 311 may extend from the circulation duct 320 to edges of both side surfaces of the machine room 300, and the ironing module S may be seated on the base bottom 311.

In addition, the ironing module S may be installed in the machine room 300 by being disposed outside the base bottom 311. That is, the base bottom 311 may have a smaller area size than the bottom surface of the machine room 300, and the ironing module S may form a remaining bottom surface of the machine room 300.

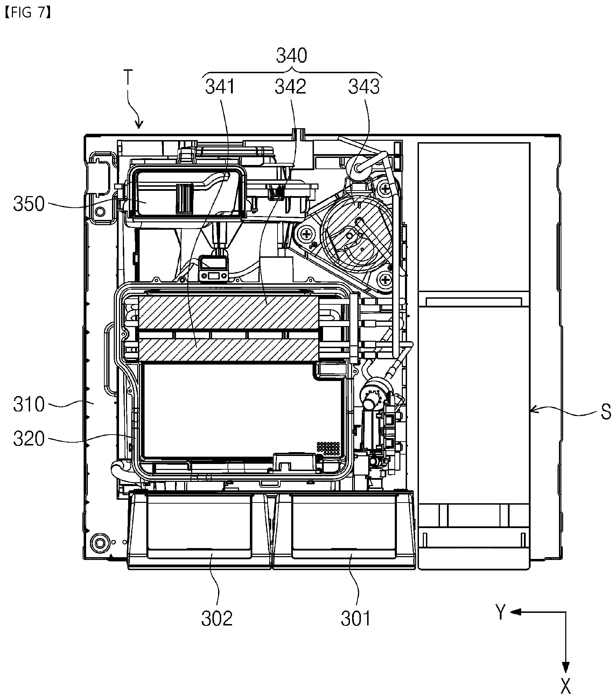

FIG. 7 shows inside of a circulation duct of a clothing treatment apparatus of the present disclosure.

The circulation duct 320 may extend upward from the base bottom to form the flow channel through which air flows.

The heat supply portion 340 may include the evaporator 341 that cools and condenses air flowing along the circulation duct 320, the compressor 343 that receives the refrigerant from the evaporator 341 and compresses and heats the refrigerant, and the condenser 342 that receives the refrigerant from the compressor 343 and heats air flowing along the circulation duct 320.

The heat supply portion 340 may further include an expansion valve that expands the refrigerant that has passed through the condenser 342 to lower a temperature of the refrigerant.

The heat supply portion 340 may include the evaporator 341 that is installed inside the circulation duct 320 and is equipped as a heat exchanger that cools and dehumidifies air that has been introduced into the circulation duct 320, the condenser 342 that is equipped as a heat exchanger that heats air that has passed through the evaporator 341 to generate hot air, the compressor 343 that supplies the refrigerant that exchanges heat with air to the condenser 342 and is disposed outside the circulation duct 320, and an expansion valve 344 that expands and cools the refrigerant that has passed through the condenser 342.

The evaporator 341 and the condenser 342 may be disposed inside the circulation duct 320, and the compressor 343 may be disposed outside the circulation duct 320.

The circulation duct 320 may include the heat exchanger installation portion 3212 that provides a space in which the evaporator 341 and the condenser 342 are installed. The heat exchanger installation portion 3212 may be defined inside the duct body 321.

The duct body 321 may have an open top surface. The condenser 343 and the evaporator 341 may be introduced via the opening of the duct body 321 and installed.

The opening of the duct body 321 may be shielded by the base cover 360, and the base cover 360 and the duct body 321 may form a flow channel through which air flows inside the circulation duct 320.

A front surface of the duct body 321 may be disposed to be spaced rearwardly apart from a front end of the base bottom 311. As a result, the base bottom 311 may secure a support surface 3111 on which at least one of the water supply tank 301, the drainage tank 302, and the outside air duct 370 described above is installed and supported.

In one example, as the duct body 321 is integrally formed with the base 310, a height of the heat exchanger installation portion 3212 may be secured greater and heights of the condenser 342 and the evaporator 341 may also be increased to that extent accordingly.

As a result, widths in the front and rear direction of the condenser 342 and the evaporator 341 may be reduced, so that the number of refrigerant pipes passing through the condenser and the evaporator may be reduced. In addition, an effect of reducing a flow loss of air passing through the condenser and the evaporator is derived.

In one example, a sum of a length of the evaporator 341 and a length of the condenser 342 may be smaller than a length of the heat exchanger installation portion 3212. Accordingly, the length in the front and rear direction of the heat exchanger installation portion 3212 may be equal to or smaller than half of the length of the duct body 321.

The blower 350 may be disposed to overlap the condenser 342 or the evaporator 341 in the front and rear direction. Therefore, air that has passed through the evaporator 341 and the condenser 342 may be introduced into the blower 350 without bending of the flow channel. That is, in a process in which air introduced into the circulation duct 320 flows to the blower 350, because there is no bending in the flow channel, the flow loss may be minimized.

A length of the ironing module S may be greater than a length of the circulation duct 320.

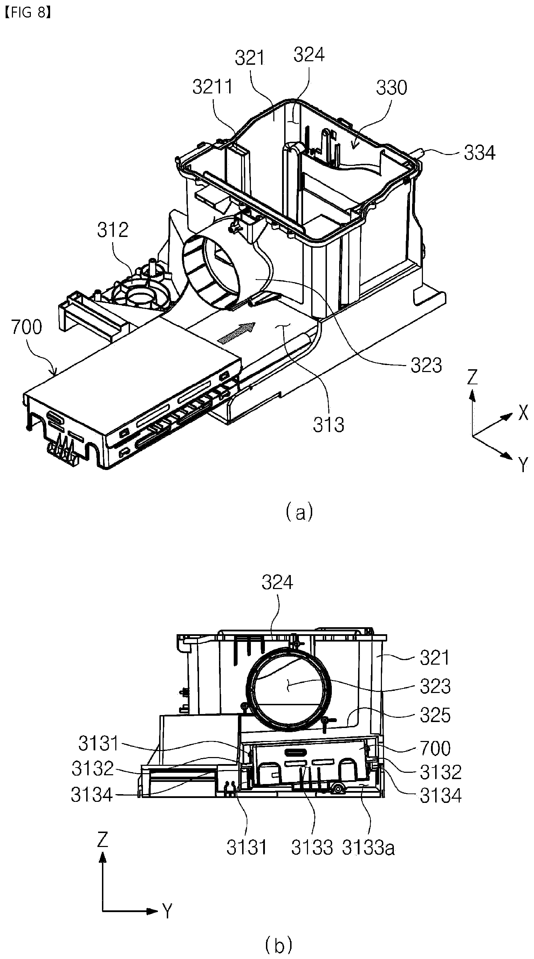

FIG. 8 shows a structure of a controller installation portion disposed on a base of a clothing treatment apparatus of the present disclosure.

The refreshing module T may further include a main controller 700 that supplies power to the heat supply portion 340 and the steam supply portion 800 or controls the heat supply portion 340 and the steam supply portion 800.

The main controller 700 may be disposed inside the machine room 300. As a result, a volume of the inner casing 200 may be secured to the maximum.

The main controller 700 may be seated on the base 310.

The main controller 700 may be disposed outside the circulation duct 320.

For example, the main controller 700 may be disposed on a left or right side of the circulation duct 320.

For example, the base 310 may be equipped with a controller installation portion 313 that defines a space in which the main controller 700 may be inserted under the circulation duct 320.

The base 310 may include the controller installation portion 313 in which the main controller 700 is installed. The controller installation portion 313 may be formed between the base bottom 311 and the circulation duct 320. The controller installation portion 313 may be formed between the base bottom 311 and a bottom surface of the circulation duct 320. The controller installation portion 313 may be formed in a duct shape with one of a front surface and a rear surface opened, under the circulation duct 320.

The main controller 700 may be connected to an external power source and supplied with the power, and may supply the power to all electronically controlled components such as the compressor 343, the steam supply portion 800, and the blower fan 353.

In addition, the main controller 700 may be equipped to control all of the electronically controlled components such as the compressor 343, the steam supply portion 800, and the blower fan 353, and may perform various courses and options for treating the laundry.

When the main controller 700 is inserted into and supported in the controller installation portion 313, vibration or impact applied to the main controller 700 may be buffered. In addition, because the main controller 700 is disposed close to the electrical components constituting the refreshing module T, occurrence of control errors such as noise may be minimized.

In addition, the steam supply is disposed on the upper side of the circulation duct 320, and the main controller 700 is disposed under the circulation duct 320. Therefore, the circulation duct 320 may be formed in a straight duct shape between the steam supply portion 800 and the main controller 700. Therefore, a flow resistance of air passing through the circulation duct 320 may be minimized.

The circulation duct 320, the outside air duct 370, the steam supply portion 800, the main controller 700, and the heat supply portion 340 may be installed in a modular manner on the base 310. Accordingly, the base 310 may be easily installed and maintained by being extended forward from and retracted rearward into the machine room 300.

(a) in FIG. 8 shows an embodiment in which the main controller 700 is installed in the controller installation portion 313.

The main controller 700 may be equipped as a PCB panel, but may not be limited thereto and may be equipped as various devices for control.

The main controller 700 may be inserted into and seated in the controller installation portion 313 disposed under the circulation duct 320. The bottom surface of the circulation duct 320 may form a top surface of the controller installation portion 313. The controller installation portion 313 may be disposed below the air discharger 323.

The controller installation portion 313 may be formed integrally with the base bottom 311. The controller installation portion 313 may be defined as a recessed space under the circulation duct during a process of molidng the circulation duct 320 on the base 310.

The main controller 700 may be slidably retracted forward into the controller installation portion 313.

A bracket 3131 may be further disposed on a surface of the main controller 700 to surround the controller. The brackets 3131 may be disposed on an upper portion and a lower portion of the controller to prevent the foreign substances from entering the controller.

In addition, the bracket 3131 may prevent a circuit board inside the main controller 700 from being damaged by heat or vibration transmitted to the main controller 700. The bracket 3131 may be made of a metal material.

(b) in FIG. 8 shows a state in which a controller is installed in a controller installation portion.

As shown in the drawing, the main controller 700 may be installed at a predetermined angle with the base bottom 311. For example, the main controller 700 may be disposed to be inclined toward the reservoir 326. Accordingly, when water leaks from the upper portion of the main controller 700, water may quickly escape the main controller 700.

The main controller 700 may include a supporter 3132 that is formed to protrude from a side surface thereof.

The controller installation portion 313 may include ribs 3134 that protrude from both side surfaces of the installation portion, respectively. The supporter 3132 of the controller may be mounted on the rib 3134.

The supporter 3132 of the controller may support an entire load of the main controller 700. When the supporter 3132 of the controller is supported on the rib 3134, the main controller 700 may be spaced apart from the base bottom 311 by a predetermined distance.

The rib 3134 may be formed integrally with the base 310. The rib 3134 may be molded together with the base 310 when the base 310 is injection-molded, and may be formed integrally with the components such as the base bottom 311, the circulation duct 320, and the like.

A protrusion 3133 may be formed protrudingly on a front surface of the main controller 700. In addition, a guide protruding rearward may be disposed on an inner surface of the controller installation portion 313. The protrusion may be coupled with the guide. The protrusion may be inserted into the guide. When the controller is introduced into the controller installation portion, the controller may be aligned at a correct location by coupling the protrusion to the guide.

In addition, locations of both side surfaces of the controller may be determined in such a way that the supporter is seated on the rib as described above. With such coupling process, the controller may be coupled at the correct location of the controller installation portion without a separate fastening member.

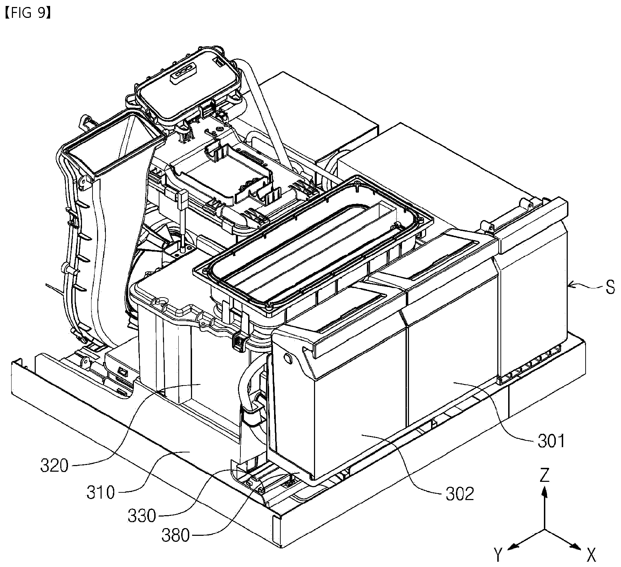

FIG. 9 shows a water supply and drainage system of a clothing treatment apparatus of the present disclosure.

The clothing treatment apparatus of the present disclosure may include the water supply tank 301 that supplies water to the steam supply portion 800 and the drainage tank 302 that collects condensed water from the circulation duct 320 or the ironing module S.

The water supply tank 301 and the drainage tank 302 should be selectively separated from each other in the machine room 300 for the user to easily fill the water supply tank 301 with water and discard water collected in the drainage tank 302.

The machine room 300 may further include a detachment portion 380 disposed in front of the refreshing module T to support the water supply tank 301 and the drainage tank 302. The detachment portion 380 may be detachably coupled to the water supply tank 301 and the drainage tank 302.

The detachment portion 380 may be formed in a plate shape to prevent the inside of the machine room 300 from being exposed.

A drainage pump 331 or the like may be disposed under the detachment portion 380, and the circulation duct 320 may be disposed behind the detachment portion 380.

A portion of the ironing module S may extend through the detachment portion 380.

The ironing module S may be disposed on one side of one of the water supply tank 301 and the drainage tank 302. The ironing module S may not be disposed between the water supply tank 301 and the drainage tank 302. As a result, the length in the front and rear direction of the ironing module S may be sufficiently secured.

The ironing module S may be disposed closer to the water supply tank 301 than to the drainage tank 302. As a result, water may be easily supplied from the water supply tank 301, heated, and delivered to the steam iron 1300, a structure of receiving water from the water supply tank 301 may be simply designed, and a volume occupied by the structure may also be minimized.

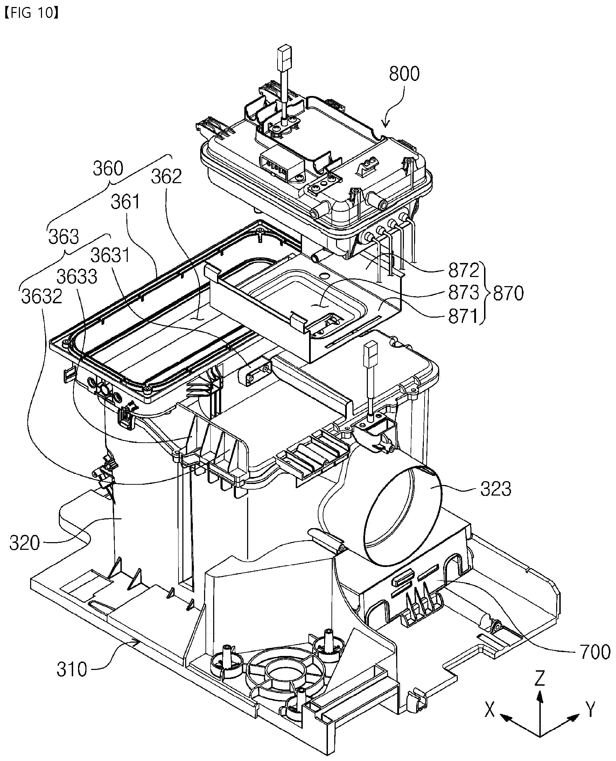

FIG. 10 shows an installation structure of a steam supply that receives water from a water supply tank.

The steam supply portion 800 may be supported by being seated on the base cover 360.

The base cover 360 may include an inflow body 361 that is coupled to the top surface of the circulation duct 320 to allow the inner casing 200 and the circulation duct 320 to be in communication with each other, and a shielding body 363 that extends from the inflow body 361 to shield the top surface of the circulation duct 320.

The inflow body 361 may be formed in a duct shape and allow the inflow hole 232 of the inner casing and the inside of the circulation duct 320 to be in communication with each other. The inflow body 361 may protrude upwardly of the shielding body 363.

The inflow body 361 may be disposed in front of the evaporator 341 so as not to face the evaporator 341 and the condenser 342.

The inflow body 361 may serve as an inflow duct that allows air in the inner casing 200 to flow to the circulation duct 320. The inflow body 361 may be equipped with the inlet 362 through which air in the inner casing 200 may pass.

The inlet 362 may be partitioned into a plurality of sections, and a filter may be inserted into and installed in one of the sections.

The shielding body 363 may include a blocking panel 3631 that blocks the evaporator 341 and the condenser 342 from being exposed to the outside and supports the steam supply portion 800, a power terminal 3633 that extends from the blocking panel 3631 or the inflow body 361 and supplies power to the steam supply portion 800, and a protective panel 3632 that accommodates therein and protects one of both side surfaces of the steam supply portion 800.

A plurality of reinforcing ribs that reinforce rigidity may be disposed on an outer surface of the protective panel 3632. This prevents vibration or impact generated in the inner casing 200 or the machine room 300 from being transmitted to the steam supply portion 800.

The steam supply portion 800 may be supported by being seated on the base cover 360.

The steam supply portion 800 may include a steam casing 810 that stores water that generates steam.

The steam supply portion 800 may further include an installation bracket 870 that may fix the steam casing 810 to the base cover 360.

The installation bracket 870 may be coupled to the base cover 360 to fix the steam casing 810.

The installation bracket 870 may include a lower panel 871 that supports a bottom surface of the steam casing 810 and side panels 872 that support both side surfaces of the steam casing 810 on the lower panel 871.

The installation bracket 870 may further include a support panel 873 that extends stepwise from the lower panel 871 and supports the bottom surface of the steam casing 810. As a result, the lower panel 871 may accommodate therein and support a portion of the bottom surface of the steam casing 810.

The compressor 343 may be disposed under the steam supply portion 800.

The installation bracket 870 may block heat generated from the compressor or heat generated from the refrigerant compressed in the compressor from being transferred to the steam supply portion 800.

The installation bracket 870 may also block fire from being transferred to the steam supply portion 800 in the event of fire occurring in the compressor 343.

In one example, the base cover 360 may include a fastener 3631 that is disposed on the shielding body 363 and detachably coupled with the steam supply portion 800. The fastener 3631 may be formed in a structure that is detachably coupled with a protruding portion protruding from a lower portion of the steam casing 810.

Therefore, even when a large amount of water is contained inside the steam casing 810, the steam casing 810 may be stably seated on the base cover 360.

In addition, because the steam casing 810 is disposed above the circulation duct 320 and has a shorter distance to the inner casing 200, condensation of steam generated in the steam casing 810 before reaching the inner casing 200 may be minimized.

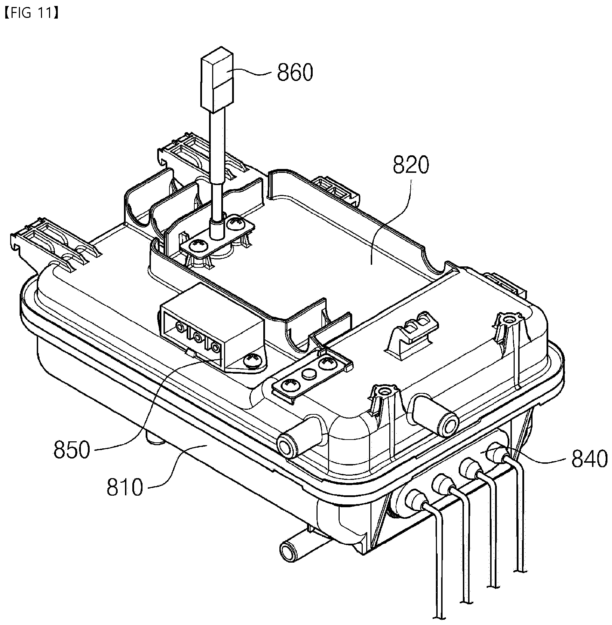

FIG. 11 shows an outer appearance of the steam supply.

The steam supply portion 800 may include the steam casing 810 that may receive and store water to generate steam, and a heater assembly 840 that is accommodated in the steam casing 810 and heats water to generate steam.

The steam casing 810 may be formed in a shape of a casing with an open top, and may accommodate the heater assembly 840 therein.

The steam supply portion 800 may further include a casing cover 820 that is coupled to the steam casing 810 to prevent the heater assembly 840 from being exposed to the outside and to prevent water from leaking.

The casing cover 820 may be equipped with a water level sensor 850 that senses a water level of the steam casing 810, and a steam sensor 860 that senses a temperature inside the steam casing 810 or senses whether steam is generated inside the steam casing 810.

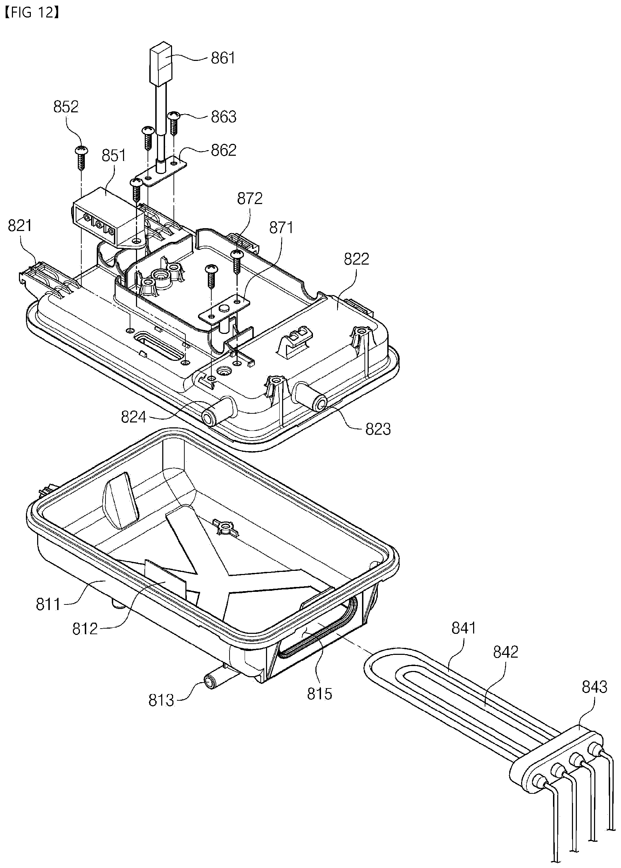

FIG. 12 shows an internal structure of a steam supply.

The steam casing 810 may include a casing body 811 that provides a space for storing water and accommodating the heater assembly 840.

The casing body 811 may be formed in a shape in which an upper portion is open. Accordingly, various parts may be easily installed inside the casing body 811.

The casing body 811 may include a heater insertion hole 815 that is defined to extend through one side of the casing body 811 and allows the heater assembly 840 to be inserted and withdrawn therethrough.

The casing body 811 may include a residual water pipe 813 that may discharge water contained inside to the outside. The residual water pipe 813 may be kept closed by a residual water stopper so as to be opened only when residual water inside the steam casing 810 is removed.

In one example, a heater fixer 812 that may support or fix the heater assembly 840 may be installed inside the casing body 811.

In one example, the steam supply portion 800 may be equipped with a recovery pipe 824 that receives water. The recovery pipe 824 may be in communication with the water supply tank 301 to receive water, and may be in communication with the water supply tank 301 via the steam nozzle 900.

The recovery pipe 824 may be equipped on the casing cover 820. As a result, water may be stored throughout the steam casing 810, and water may be prevented from flowing back via the recovery pipe 824.

The steam supply portion 800 may be equipped with a steam pipe 823 that discharges steam generated by the operation of the heater assembly 840 to the outside. The steam pipe 823 may also be disposed on an upper portion of the casing cover 820 to block water from being discharged into the steam pipe 823. The steam pipe 823 may be connected to the steam nozzle 900.

The water level sensor 850 may include a sensor body 851 having at least one contact protrusion that is inserted into a water level sensor hole 854 and is immersed in water to sense the water level, and a fixing member 852 that fixes the sensor body 851 to the casing cover 820.

The steam sensor 860 may include a sensor 861 that senses a temperature or a pressure change inside the steam casing 810, a support plate 862 that supports the sensor 861, and a fastening member 862 that fixes the support plate 862 to the casing cover 820.

In one example, the steam supply portion 800 may further include a fixing plate 871 that is coupled to the casing cover 820 to fix the heater assembly 840, and a coupling member 872 that fixes the fixing plate 871 to the casing cover 820.

The casing cover 820 may further include a cover hook 821 that extends forward and is able to be coupled to the base cover 360.

The heater assembly 840 may be inserted into the heater insertion hole 815 to be accommodated in the steam casing 810 and may receive the power and heat water.

The heater assembly 840 may be equipped as a sheath heater or the like, and may be controlled such that operation and stopping may be repeated by the main controller 700.

The heater assembly 840 may include a first heater 841 that receives first power to heat water, and a second heater 842 that receives power smaller than the first power to heat water. As a result, the first heater 841 may heat a larger amount of water than the second heater 842 to generate more steam.

The first heater 841 and the second heater 842 may share a maximum heater power capacity allowed for the heater assembly 840 in the clothing treatment apparatus. That is, when the first heater 841 is provided to consume a portion of the maximum heater power capacity, the second heater 842 may be provided to consume the remainder of the maximum heater power capacity.

For example, when general maximum heater power capacity that may be allowed for the heater assembly 840 is 1500W, the second heater 842 may be provided to consume 600W, and the first heater 841 may be provided to consume 880W. The remaining 20W may be kept as a margin for an error or the like.

In one example, the heater assembly 840 may include three or more heaters. For example, the heater assembly 840 may include the first heater 841, the second heater 842, and a third heater 843, and the first heater 841, the second heater 842, and the third heater 843 may share the maximum heater power capacity.

Hereinafter, a description will be made based on the heater assembly 840 being composed of the first heater 841 and the second heater 842.

The first heater 841 and the second heater 842 may be formed as U-shaped metal pipes.

The heater assembly 840 may further include a heater sealer 843 that may fix the first heater 841 and the second heater 842 and seal a heater through-hole 8111.

The first heater 841 and the second heater 842 may be disposed at the same vertical level. Accordingly, the first heater 841 and the second heater 842 may heat water of the same water level to generate steam.

Therefore, the main controller 700 may selectively use the first heater 841 and the second heater 842 to adjust an amount of steam generated and an amount of power consumed.

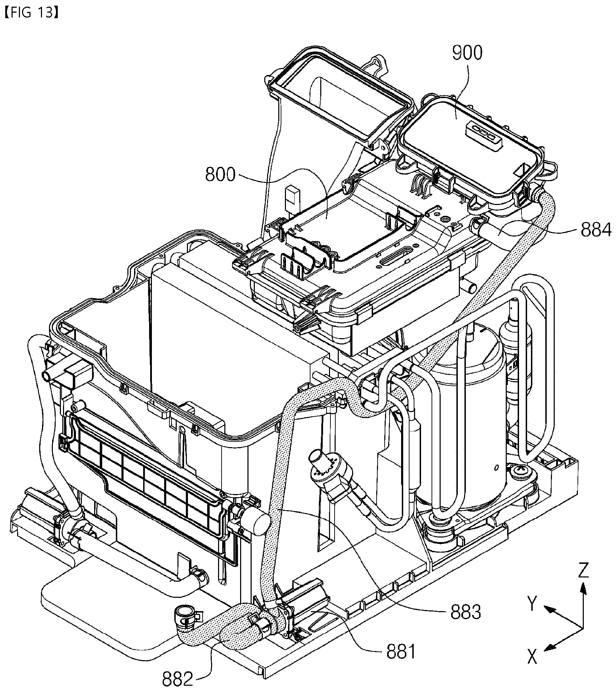

FIG. 13 shows a structure of supplying water to the steam supply.

Steam generated inside the steam casing 810 may be directly supplied into the inner casing 200 via a steam pipe 823.

However, when steam is directly supplied into the inner casing 200 via the steam pipe 823, there is a concern that water contained in the steam casing 810 may also be discharged into the inner casing 200. In addition, there is a concern that water heated in the steam casing 810 may heat the bottom surface of the inner casing 200, causing thermal damage to the inner casing 200.

To prevent such problem, the steam supply portion 800 of the present disclosure may further include the steam nozzle 900 that receives steam generated in the steam casing 810 and supplies steam to the inner casing 200.

The steam nozzle 900 may be disposed to be spaced apart from the steam casing 810, and may receive only steam formed in the steam casing 810 and not receive water contained in the steam casing 810.

For example, the steam nozzle 900 may be disposed above the steam casing 810 and may be connected thereto via the steam pipe 823. Accordingly, steam generated in the steam casing 810 may rise because of a density difference and be supplied to the steam nozzle 900 along the steam pipe 823, and water contained in the steam casing 810 may not be introduced into the steam pipe 823 or the steam nozzle 900 because of gravity.

The steam nozzle 900 may be disposed between the bottom surface of the inner casing 200 and the steam casing 810, and may be in communication with the steam hole 233.

In one example, the steam supply portion 800 of the present disclosure may receive water from the water supply tank 301 and generate steam.

To this end, the clothing treatment apparatus of the present disclosure may include a water supply 880 that supplies water contained in the water supply tank 301 to the steam supply portion 800.

The water supply 880 may include a water supply pump 881 that provides a pressure to supply water supplied to the water supply tank 301 to the steam supply portion 800 or the steam nozzle 900, a supply pipe 882 that is in communication with the water supply tank 301 and delivers water to the water supply pump 881, and a connection pipe 883 that connects the water supply pump 881 with the steam nozzle 900.

The supply pipe 882 may be formed as a hose connecting the water supply tank 301 with the water supply pump 881, and the connection pipe 883 may be formed as a hose connected to the water supply pump 881.

The clothing treatment apparatus of the present disclosure may supply water contained in the water supply tank 301 to the steam nozzle 900 rather than to the steam casing 810.

The water supply pump 881 may not be directly connected to the steam casing 810.

The steam nozzle 900 may receive water via the connection pipe 883 and then supply water to the steam supply portion 800 via a water supply pipe 884. The water supply pipe 884 may be connected to a recovery pipe 824.

Because the steam nozzle 900 is disposed above the steam casing 810, water supplied to the steam nozzle 900 may be automatically supplied to the steam casing 810 via the water supply pipe 884.

The steam pipe 823 through which steam is introduced into the steam nozzle 900 and the recovery pipe 824 through which water is discharged from the steam nozzle 900 may be formed as separate flow channels. Therefore, water supplied from the steam nozzle 900 may be prevented from interfering with flow of steam supplied from the steam casing 810.

In one example, the water supply pipe 884 may be formed in a U-shape. That is, a middle area of the water supply pipe 884 may be disposed at a lower vertical level than both ends thereof. As a result, a certain amount of water, like a water trap, may be accumulated in the water supply pipe 884, thereby preventing steam or water supplied from the steam casing 810 from being re-introduced via the water supply pipe 884.

In addition, because the steam casing 810 is not directly connected to the water supply pump 881, a possibility that water introduced into the steam casing 810 may flow back into the water supply pump 881 may be blocked.

In addition, a large amount of water may be supplied to the steam nozzle 900 at a considerable pressure via the water supply pump 881. As a result, foreign substances, bacteria, or the like accumulated or growing in the steam nozzle 900 may be washed away by water supplied to the steam nozzle 900 and may go down into the steam casing 810. Therefore, the steam nozzle 900 may always be maintained in a clean state.

In one example, when steam supplied from the steam nozzle 900 is condensed, condensed water may be re-introduced into the steam casing 810 via the water supply pipe 884. That is, condensate generated from the steam nozzle 900 may be recovered into the steam casing 810 in the same direction as a flow direction of water supplied from the water supply pump 881, and may be recycled as water that generates steam. Therefore, the clothing treatment apparatus of the present disclosure may prevent a water level of the water supply tank 301 from rapidly decreasing and also prevent waste of water.

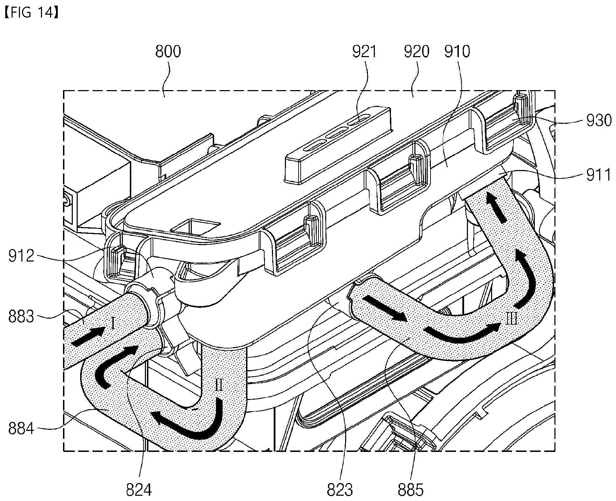

FIG. 14 shows a flow channel structure of the steam nozzle.

The steam nozzle 900 may include a supply container 910 that may receive steam from the steam casing 810 or receive water from the water supply pump 881 and accommodate the same therein.

The supply container 910 may be formed in a casing shape having an internal space that may receive and accommodate therein water or steam.

The connection pipe 883 may be connected to the supply container 910 at a location higher than a bottom surface of the supply container 910. For example, the connection pipe 883 may be coupled to a side surface of the supply container 910. As a result, water supplied to the supply container 910 may be prevented from flowing back to the connection pipe 883.

The connection pipe 883 may be connected to the supply container 910 at a location higher than the recovery pipe 824. For example, the recovery pipe 824 may be disposed such that one end is coupled to the bottom surface of the supply container 910 and the other end is coupled to the steam casing 810. As a result, water supplied to the water supply pipe 884 may be automatically introduced into the recovery pipe 824. In addition, the recovery pipe 824 may be formed in a U-shape so as to accommodate a certain amount of water inside.

In one example, the recovery pipe 824 may be disposed adjacent to the water supply pipe 884. As a result, water supplied to the water supply pipe 884 may be quickly introduced into the steam casing 810 without remaining in the supply container 910.

In one example, the steam pipe 823 may have one end coupled to a lower portion of the supply container 910 and the other end coupled to an upper end of the steam casing 810 or the casing cover 820. As a result, steam generated in the steam casing 810 may be automatically supplied to the supply container 910 because of a density difference.

The steam pipe 823 may be connected to a location on the supply container 910 higher than the recovery pipe 824. In addition, the steam pipe 823 may be disposed further from the water supply pipe 884 than the recovery pipe 824.

As a result, water supplied to the supply container 910 may be supplied more to the recovery pipe 824 without flowing back to the steam pipe 823.

The steam pipe 823 may be coupled to the bottom surface of the supply container 910 via a steam hose 885.

In the bottom surface of the supply container 910, a portion where the recovery pipe 824 is disposed may be lower and a portion coupled to the steam hose 885 may be higher. To this end, the bottom surface of the supply container 910 may be formed with a step or may be inclined.

The steam nozzle 900 may further include a container cover 920 coupled to an upper portion of the supply container 910. The container cover 920 may include a steam spray hole 921 defined through an upper portion thereof. The steam spray hole 921 may be in communication with the inside of the inner casing 200.

The supply container 910 may be formed in a box shape with an open top, and the container cover 920 may shield the top of the supply container 910.

Steam supplied via the steam pipe 823 may be discharged via the steam injection hole 921 and supplied into the inner casing 200.

The steam pipe 823 may be disposed between the steam spray hole 921 and an inner surface of the supply container 910. The steam spray hole 921 may be disposed between the water supply pipe 884 and the steam pipe 823, and may be disposed between the recovery pipe 824 and the steam pipe 823. As a result, steam that has not been discharged via the steam spray hole 921 and is condensed may not remain inside the supply container 910 and may be discharged via the recovery pipe 824.

The steam spray hole 921 may be disposed at a center based on a width direction of the container cover 920.

The supply container 910 may have the open top, and thus, may be advantageous in installing various shapes or structures inside, and the container cover 920 may be advantageous in installing various structures at a lower portion.

The steam nozzle 900 may include a fastener 930 that couples the container cover 920 with the supply container 910.

Based on the steam spray hole 921, the water supply pipe 884 and the recovery pipe 824 are disposed on one side of the supply container 910, and the steam pipe 823 is disposed on the other side of the supply container 910.

That is, the water supply pipe 884 and the recovery pipe 824 may be disposed adjacent to each other, and the steam pipe 823 may be disposed further from the water supply pipe 884 than the recovery pipe 824.

Accordingly, water flowing to the water supply pipe 884 may be supplied to the supply container 910 along a direction I. Water supplied to the supply container 910 may be immediately discharged along a direction II to the recovery pipe 824 and supplied to the steam casing 810. Steam supplied from the steam pipe 823 may be supplied to the supply container 910 along a direction III and discharged to the steam spray hole 921, and condensed water may be discharged along the direction II to the recovery pipe 824 and re-supplied to the steam casing 810.

The supply container 910 may further include an inflow pipe 912 that is coupled to the water supply pipe 884 to receive water, and a hose pipe 911 that is connected to the steam hose 885 to receive steam.

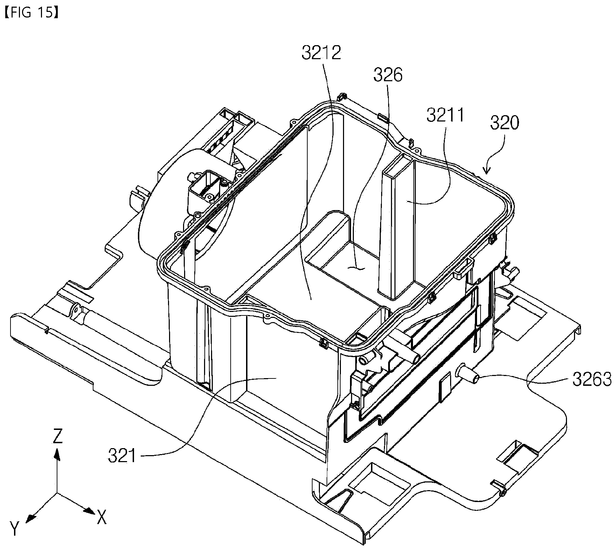

FIG. 15 shows a drainage structure of a clothing treatment apparatus of the present disclosure.

In the clothing treatment apparatus of the present disclosure, when the compressor 343 and the blower fan 353 are operated, air supplied from the outside of the cabinet 100 and air supplied from the inner casing 200 are cooled while passing through the evaporator 341, and water vapor contained in the air is condensed.

Water condensed in the evaporator 341 may accumulate on the bottom surface of the circulation duct 320.

The clothing treatment apparatus of the present disclosure may include a reservoir 326 defined as a portion of the bottom surface of the duct body 321 is recessed to collect condensate condensed in the evaporator 341.

The reservoir 326 is a space defined as the portion of the bottom surface of the duct body 321 is recessed, and is able to form one side surface of a controller installation portion 313.

The reservoir 326 may be defined to be recessed downward from the bottom surface of the circulation duct 320.

The reservoir 326 may be formed integrally with the circulation duct 320. The reservoir 326 may be formed by forming the portion of the bottom surface of the circulation duct 320 to be recessed while injection-molding the circulation duct 320 onto the base 310.

The reservoir 326 may have at least a portion of a top surface thereof disposed parallel to the heat exchanger installation portion 3212.

The base 310 may include a guide pipe 3263 that discharges water collected in the reservoir 326 to the outside.

The guide pipe 3263 may protrude from a lower portion of the reservoir 326 to the outside of the circulation duct 320. The guide pipe 3263 may discharge water stored in the reservoir to the outside of the base. This may prevent water collected in the reservoir 326 from decaying or flowing back to the bottom surface of the circulation duct 320.

The circulation duct 320 may include a partition wall 3211 that extends from an inner surface of the duct body 321. The partition wall 3211 may protrude inwardly from an inner wall of the circulation duct 320, or an outer wall of the circulation duct 320 may be recessed inward and protrude inward. The partition wall 3211 may guide a location where the heat exchanger 341 and 343 is installed, and may prevent air entering the heat exchanger from passing by bypassing the heat exchanger. The partition wall 3211 may be disposed in the reservoir 326.

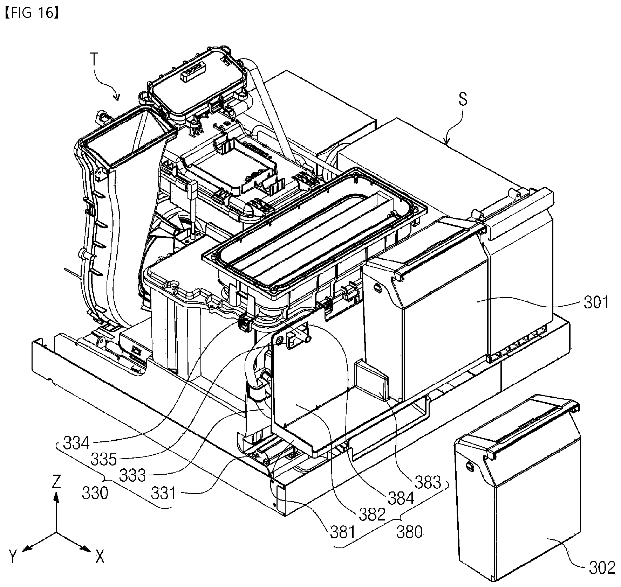

FIG. 16 shows a drainage tank installation structure of a clothing treatment apparatus of the present disclosure.

The drainage tank 302 may be formed in a shape of a box that stores water.

The drainage tank 302 may include a supply hole for receiving water defined in a rear surface, and the supply hole may be defined closer to an upper end than to the bottom surface of the drainage tank 302.

The detachment portion 380 may include a load support 381 on which at least one of the drainage tank 302, the water supply tank 301, and the ironing module S may be supported, and a detachment support 382 disposed on the load support 381 in front of the circulation duct 320 and on which the drainage tank 302 and the water supply tank 301 may be seated.

The load support 381 may be formed in a plate shape to support bottom surfaces of the water supply tank 301 and the drainage tank 302, and the detachment support 382 may also be formed in a plate shape to support rear surfaces of the water supply tank 301 and the drainage tank 302.

The detachment portion 380 may include a detachment separator 383 protruding from the load support 381 so as to be disposed between the water supply tank 301 and the drainage tank 302. The detachment separator 383 may separate the water supply tank 301 and the drainage tank 302 from each other by a predetermined spacing. As a result, when one of the water supply tank 301 and the drainage tank 302 is withdrawn, the other of the water supply tank 301 and the drainage tank 302 may be prevented from being withdrawn arbitrarily.

The clothing treatment apparatus of the present disclosure may further include a drainage 330 that collects water condensed in the circulation duct 320 into the drainage tank 302.

The drainage 330 may include the drainage pump 331 that receives water from the guide pipe 3263 and a drainage hose 333 that receives water from the drainage pump 331 and guides water to the drainage tank 302.

The drainage 330 of the present disclosure may further include a discharge pipe 334 that allows the drainage hose 333 and the drainage tank 302 to be in communication with each other. The discharge pipe 334 may receive water from the drainage hose 333 and guide water into the drainage tank 302.

In one example, the discharge pipe 334 may be directly connected to the drainage hose 333 or may be formed integrally with the drainage hose 333.

However, the clothing treatment apparatus of the present disclosure may install the discharge pipe 334 in the circulation duct 320 such that water may be continuously supplied even when a water level inside the drainage tank 302 reaches a full level, and the circulation duct 320 may be provided to receive water flowing back from the discharge pipe 334 or the drainage tank 302 again therein.

As a result, the circulation duct 320 and the reservoir 326 may temporarily perform the role of the drainage tank 302 to expand a water storage capacity. As a result, water may be prevented from overflowing out of the drainage tank 302.

For example, the detachment portion 380 may include a detachment communication portion 384 that is defined to extend through the detachment support 382 so as to allow the drainage hose 333 and the drainage tank 302 to be in communication with each other.

The detachment communication portion 384 may be defined in an area facing the supply hole defined in the drainage tank when the drainage tank 302 is seated on the load support 381.

The discharge pipe 334 may extend from the circulation duct 320 or may extend from the detachment portion 380. The discharge pipe 334 may be formed as an injection-molded product. Accordingly, the discharge pipe 334 may stably fix a distal end of the drainage hose 333 and prevent the drainage hose 333 from being bent abruptly.

In addition, the discharge pipe 334 may be formed as an injection-molded product in a shape of a pipe fixed to the circulation duct 320 or the load support 381, so that an installation location thereof may always be fixed. Accordingly, a distal end of the discharge pipe 334 and the supply hole defined in the drainage tank 302 are always positioned in correct locations, so that condensate supplied from the drainage pump 331 may be stably introduced into the drainage tank 302.

The clothing treatment apparatus of the present disclosure may further include a backflow portion 335 that allows water contained inside the drainage tank 302 to flow back into the circulation duct 320 or the drainage 330.

The backflow portion 335 may be formed under the discharge pipe 334 and may allow the circulation duct 320 and the inside of the drainage tank 302 to be in communication with each other. The backflow portion 335 may receive water inside the drainage tank 302 and guide water into the circulation duct 320.

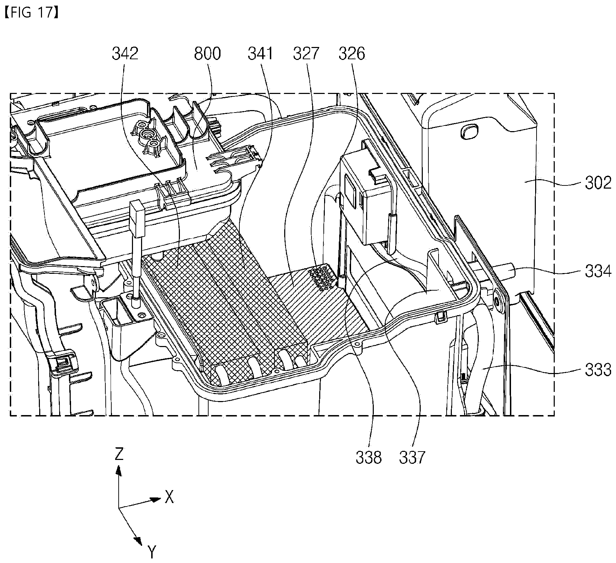

FIG. 17 shows in detail a structure of a backflow portion of a clothing treatment apparatus of the present disclosure.

The circulation duct 320 may have the evaporator 341 and the condenser 342 disposed therein. In this regard, the evaporator 341 may be disposed in front of the condenser 342 in the circulation duct 320.

The backflow portion 335 may be defined in a front surface of the circulation duct 320. In this regard, when water introduced into the circulation duct 320 from the backflow portion 335 comes into contact with the evaporator 341 or the like, the evaporator 341 may be unnecessarily corroded or contaminated and an efficiency of the evaporator 341 in cooling air may decrease.

Therefore, to prevent water supplied from the backflow portion 335 from coming into contact with the evaporator 341 along air directed toward the evaporator 341, the evaporator 341 may be disposed closer to a rear side of the circulation duct 320 than the backflow portion 335.

The backflow portion 335 or the discharge pipe 334 may be exposed forward of the circulation duct 320 by extending through the detachment communication portion 384. When the drainage tank 302 is seated on the detachment support 382, a distal end of the backflow portion 335 or the discharge pipe 334 may be inserted into the drainage tank 302.

As a result, water discharged out of the circulation duct 320 via the drainage pump 331 may be collected in the drainage tank 302 along the drainage hose 333 and the discharge pipe 334.

When the water level of the drainage tank 302 reaches the discharge pipe 334 or the backflow portion 335, water overflowing out of the backflow portion 335 may be introduced into the circulation duct 320 again along the backflow portion 335 and be prevented from leaking out of the machine room 300.

A guide flow channel 338 may extend along a width direction of the front surface of the circulation duct 320. One end of the guide flow channel 338 may be disposed to be in communication with the backflow portion 335, and the other end thereof may be disposed above the reservoir 326.

In addition, the circulation duct 320 may further include a blocking wall 337 that guides water introduced from the backflow portion 335 to the guide flow channel 338.

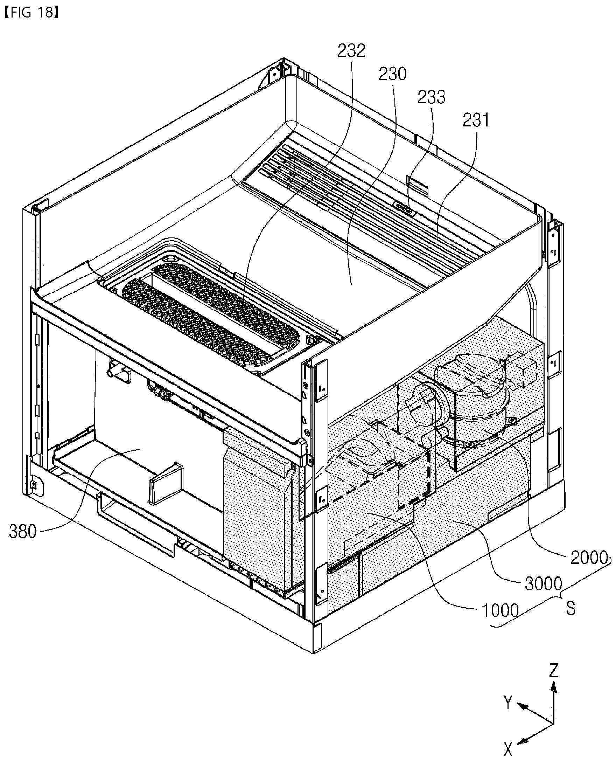

FIG. 18 shows a lower configuration of a bottom surface of an inner casing.

The bottom surface 230 may introduce air inside the inner casing 200 into the circulation duct 320 via the inflow hole 232, and may receive air dehumidified and heated while passing through the circulation duct 320, via the exhaust hole 231. In addition, steam supplied from the steam supply portion 800 may be received via the steam hole 233.

However, the bottom surface 230 except for the through-holes may block air, moisture, and foreign substances inside the inner casing 200 from flowing out into the machine room 300.

The detachment portion 380 may be disposed in a front side of the machine room 300 to prevent external air, moisture, and foreign substances from being introduced into the machine room 300.

The clothing treatment apparatus of the present disclosure may install the ironing module S in the machine room 300. As a result, the ironing module S may be prevented from being exposed to air, moisture, and the foreign substances inside the inner casing 200.

In addition, the machine room 300 may have the entire components disposed under the bottom surface 230, so that the machine room 300 may be disposed and installed independently of the inner casing 200. As a result, the machine room 300 may be manufactured as a module and installed in the cabinet 100.

The ironing module S may be installed in the machine room 300 in a modular manner. Therefore, the ironing module S may also be selectively installed in the machine room 300 without affecting the internal configurations of the machine room 300 and the cabinet 100.

Therefore, even when the clothing treatment apparatus of the present disclosure is a product that does not have the ironing module S, the ironing module S may be installed in the machine room 300 in the future.

The ironing module S may be disposed to one of both side surfaces of the machine room 300. The ironing module S may minimize interference with existing components such as the circulation duct 320.

The ironing module S may include a storage module 1000 that may store the steam iron 1300, which supplies at least one of heat and steam to the laundry, inside the machine room 300, and a heating module 2000, which generates at least one of heat and steam to be supplied to the steam iron 1300.

The heating module 2000 may be disposed behind the storage module 1000, and the storage module 1000 may be exposed forward of the machine room 300. As a result, the steam iron 1300 may be withdrawn forward of the machine room 300 and be accessible to the laundry.

The detachment portion 380 may have a passage surface that supports the steam iron 1300 so as to be withdrawn forward from the inside of the machine room 300.

The ironing module S may further include a base module 3000 that supports the storage module 1000 and the heating module 2000. The base module 3000 may provide a space in which the storage module 1000 and the heating module 2000 are installed, and may fix locations and a connected state of the storage module 1000 and the heating module 2000.

In addition, the base module 3000 may be provided to position the storage module 1000 and the heating module 2000 above the base 310 or the bottom surface of the cabinet by a predetermined vertical dimension or more.

As a result, condensed water or residual water in the storage module 1000 and the heating module 2000 may be automatically discharged by gravity, and the storage module 1000 and the heating module 2000 may be prevented from being damaged by vibration or impact.

The storage module 1000 and the heating module 2000 may be mounted on the base module 3000 and installed inside the machine room 300.

In one example, when the storage module 1000 and the heating module 2000 may be stably installed in the machine room 300, the base module 3000 may be omitted.

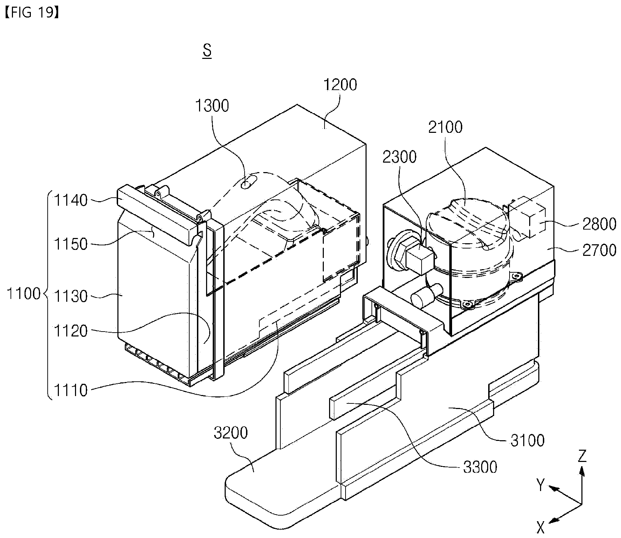

FIG. 19 shows a structure of the ironing module in detail.

The iron module S of the clothing treatment apparatus of the present disclosure may include the storage module 1000 in which the steam iron 1300 that may directly supply heat and steam to the surface of the laundry is stored, the heating module 2000 that supplies heat and steam to the steam iron 1300, and the base module 3000 that supports the storage module 1000 and the heating module 2000.

The storage module 1000, the heating module 2000, and the base module 3000 may be manufactured integrally, or may be manufactured separately and then coupled and fixed to each other.

Hereinafter, a description will be made based on the storage module 1000, the heating module 2000, and the base module 3000 being provided as independent modules, but it is not excluded that they are manufactured integrally.

The storage module 1000, the heating module 2000, and the base module 3000 may be manufactured independently of each other and installed in the machine room 300. Accordingly, the storage module 1000, the heating module 2000, and the base module 3000, which have volumes smaller than that of the entire ironing module S, may be installed sequentially in the machine room 300, so that a work of installing the ironing module S in the machine room 300 may be easy.

In addition, when one of the storage module 1000, the heating module 2000, and the base module 3000 is damaged or defective, it is required to replace or repair only the specific module, so that maintenance and repair of the ironing module S may be easy.

The storage module 1000 may include the steam iron 1300 that may approach the laundry and supply at least one of heat and steam to the laundry.

The steam iron 1300 may have a surface, facing the laundry, made of a material that may transmit heat, so that when the steam iron 1300 comes into contact with the laundry, steam iron 1300 may remove the wrinkles from the laundry.

In addition, the steam iron 1300 may include a spray hole through which steam is sprayed onto the surface thereof facing the laundry, so that high-temperature steam may be supplied to the surface of the laundry to not only remove the wrinkles from the laundry, but also perform a refreshing process such as sterilization and deodorization of the laundry.

The steam iron 1300 may generate heat and steam by being supplied with power on its own, but may receive at least one of heat and steam from the heating module 2000 and transmit the same.

When the steam iron 1300 is equipped to receive at least one of heat and steam, a weight and a volume of the steam iron 1300 may be reduced, so that the user may easily lift the steam iron 1300 up to the surface of the laundry hung on the hanger or the fixer.

The storage module 1000 may further include the housing 1200 that accommodates the steam iron 1300 therein. The housing 1200 may be formed in a casing shape that has an accommodating space for storing the steam iron 1300 inside.

The housing 1200 may serve to store the steam iron 1300 inside the machine room 300, and may perform a role of varying a location where the steam iron 1300 is arbitrarily stored inside the machine room 300.

A width of the housing 1200 may be greater than a width of the steam iron 1300, and a length of the housing 1200 may be greater than a length of the steam iron 1300.

For example, the width of the housing 1200 may be about 1 to 3cm greater than the width of the steam iron 1300. However, the width of the housing 1200 may not be greater than the width of the steam iron 1300 by 5cm or more. This may prevent the steam iron 1300 from vibrating or moving in the left and right direction inside the housing 1200.

The housing 1200 may have a length in the front and rear direction greater than a width in the left and right direction, and may have a height in the vertical direction greater than the width in the left and right direction. Accordingly, the steam iron 1300 may be stably inserted into and stored inside the storage box 1200.

The housing 1200 may include the housing body 1210 that accommodates the steam iron 1300 therein, and the open surface 1220 defined in the front surface of the housing body 1210 and from which the steam iron 1300 may be withdrawn.

The housing body 1210 may accommodate the steam iron 1300 therein so as to block the steam iron 1300 from being exposed to an upper side, both side surfaces, and a rear side of the machine room.

The housing body 1210 may accommodate therein and withdraw the steam iron 1300 only through the open surface 1220. As a result, the steam iron 1300 may be exposed only forward of the machine room 300, and may be prevented from being exposed or withdrawn in other directions.

The open surface 1220 may open the entire front surface of the housing body 1210. When the steam iron 1300 is able to be withdrawn and inserted, only a portion of the front surface of the housing body 1210 may be opened.

The storage module 1000 may further include an withdrawal portion 1100 that exposes the steam iron 1300 forward of the housing 1200.

The withdrawal portion 1100 may support the steam iron 1300 inside the housing 1200. The steam iron 1300 may be seated on the withdrawal portion 1100 and stored inside the housing 1200.

The withdrawal portion 1100 may expose at least a portion of the steam iron 1300 stored inside the storage box 1200 to the outside of the housing 1200 The withdrawal portion 1100 may withdraw the steam iron 1300 forward of the housing 1200 and expose the steam iron 1300 forward of the machine room 300 or the detachment portion 380. Accordingly, even when the steam iron 1300 is stored in the housing 1200, the steam iron 1300 may be easily withdrawn out of the machine room 300.

The withdrawal portion 1100 may be of a drawer type that is withdrawn forward from the housing 1200, or may be of a hatch type that is opened by pivoting forward at a front side of the housing 1200.

The withdrawal portion 1100 may be equipped as any component as long as it is able to selectively expose the steam iron 1300 forward of the housing 1200 while storing the steam iron 1300 inside the housing 1200.

The withdrawal portion 1100 may include a support portion 1120 on which the steam iron 1300 is mounted, and a cover 1130 disposed in front of the support portion 1120 to shield the open surface 1220.

The support portion 1120 may be accommodated in the housing 1200 and fix the steam iron 1300. The support portion 1120 may be formed in a casing shape that accommodates the steam iron 1300 therein, or may be formed in a plate shape that supports a lower portion of the steam iron 1300. The support portion 1120 may be provided as a drawer accommodated in the housing body 1210 and retracted and withdrawn via the open surface 1220.

When the support portion 1120 is withdrawn from the open surface 1220, the steam iron 1300 mounted on the support portion 1120 may be withdrawn forward of the housing 1200, and when the support portion 1120 is inserted into the open surface 1220, the steam iron 1300 mounted on the support portion 1120 may be completely accommodated in the housing 1200.

The withdrawal portion 1100 may further include a cover panel 1130 that extends from a front side of the support portion 1120 and shields the open surface 1220. The cover panel 1130 may prevent the inside of the housing body 1210 from being exposed to the outside via the open surface 1220. As a result, not only the steam iron 1300 accommodated in the housing body 1210, but also an entirety of the inside of the ironing module S may be prevented from being contaminated with foreign substances, steam, or air.

The cover panel 1130 may shield a through-hole defined to allow the withdrawal portion 1100 to be withdrawn from the detachment portion 380. As a result, the inside of the machine room 300 may be prevented from being exposed to the outside because of the through-hole, and the foreign substances, steam, or air may be prevented from being introduced into the machine room 300.

An area size of the cover panel 1130 may be greater than that of a front surface of the support portion 1120. The area size and a shape of the cover panel 1130 may be an area size and a shape that may completely shield a space where the ironing module S is exposed in the detachment portion 380.

A width of the cover panel 1130 may be the same as a width of the support portion 1120 or a width of the open surface 1220, and a height of the cover panel 1130 may be greater than a height of the support portion 1120 and the same as a height of the open surface 1220.

A recessed portion 1150 that is recessed inward and an withdrawal portion handle 1140 disposed on the recessed portion 1150 may be disposed at an upper portion of the cover panel 1130.

The withdrawal portion 1100 may include a guide groove 1110 at a lower portion of the support portion 1120 to guide the support portion 1120 to slide in the front and rear direction without moving in the left and right direction.

The guide groove 1110 may be engaged with a rail disposed inside the storage body 1210 or a rail 3300 disposed in the base module 3000 and inserted into the housing body 1210. When the guide groove 1110 moves forward and rearward along the rail 3300, the entire withdrawal portion 1100 may be withdrawn from or retracted via the open surface 1220.

The storage body 1210 may have an insertion hole defined in a lower portion of a rear surface thereof into which the rail 3300 may be inserted. In addition, the storage body 1210 may include an open hole through which the cable 1400 extending from the heating module 2000 may pass through the rear surface.

The insertion hole and the open hole may be integrally defined.

However, the steam iron 1300 may be prevented from being withdrawn or exposed via the insertion hole and the open hole. For example, the insertion hole and the open hole may be defined lower than the support portion 1120 or the steam iron 1300.

The heating module 2000 may include a heat generator 2100 that supplies heat and steam to the steam iron 1300. The heat generator 2100 may receive power, heat a wire, and transmit heat to the steam iron 1300 via the cable 1400, or heat water to generate steam and transmit steam to the steam iron 1300 via the cable 1400.

For example, the heat generator 2100 may be equipped as the steam generator that receives power and water to generate steam.

The steam generator 2100 may be equipped separately from the steam supply portion 800 seated in the machine room 300. The steam generator 2100 may receive water and power and heat water to generate steam.

In addition, the steam generator 2100 may supply steam up to the steam iron 1300, and generate steam of a higher pressure than the steam supply portion 800 such that steam supplied up to the steam iron 1300 may be sprayed onto the laundry.

The steam generator 2100 may be coupled with a connector 2800 that receives power and may include a pressure sensor that senses the pressure of steam generated inside.

The steam generator 2100 may be equipped as any component as long as it is able to receive water and heat steam. The steam generator 2100 may include a steam container forming an outer appearance thereof and a steam heater that is disposed inside the steam container and heats water.

In addition, the heating module 2000 may be connected to a high-pressure pump 2200 that increases a pressure inside the steam generator 2100 to induce the generation of high-pressure steam.

The heating module 2000, unlike the storage module 1000, may be prevented from being withdrawn from the machine room 300. That is, when the heating module 2000 is installed in the machine room 300, the heating module 2000 may be fixed inside the machine room 300. For example, the heating module 2000 may be mounted on and fixed on the base module 3000.

Entire components of the heating module 2000 may be positioned behind the storage module 1000. As a result, the exposure of the storage module 1000 forward of the machine room 300 and the forward withdrawal and the rearward insertion of the steam iron 1300 from and into the machine room may not be hindered. In addition, the storage module 1000 may be prevented from being exposed rearward of the machine room 300.

The heating module 2000 may be mounted and fixed on the base module 3000 or the base 310. For the entire components of the heating module 2000 to be stably installed, an entirety of the heating module 2000 may be accommodated in a heating casing 2700. The heating casing 2700 may have an open surface in a front surface thereof through which the cable 1400 may pass, and may be formed in a box shape with both side surfaces and a top surface not exposed to the outside.

Most of the components of the heating module 2000 may be installed inside the heating casing 2700. Therefore, maintenance and repair of the heating module 2000 may be easy.

The base module 3000 may include a base panel 3200 disposed in parallel with the base 310, and a seating panel 3100 extended from the base panel 3200 and on which the storage module 1000 and the heating module 2000 are seated.

The base panel 3200 may be formed integrally with the base 310.

However, when the ironing module S is provided as the module independent of the circulation duct 320, the base panel 3200 may be formed separately from the base 310.

The seating panel 3100 may fix the lower portions of the storage module 1000 and the heating module 2000, but space the storage module 1000 and the heating module 2000 apart from the base panel 3200 by a certain vertical dimension.

As a result, a space in which the water supply structure connected to the cable 1400 or the heating module 2000 to supply water to the heating module 2000 and the drainage structure to discharge water from the heating module 2000 may be installed between the storage module 1000, the heating module 2000, and the base panel 3200.

Furthermore, because the storage module 1000 and the heating module 2000 are disposed above the drainage structure because of the seating panel 3100, condensate or residual water generated in the storage module 1000 and the heating module 2000 may be guided to the drainage structure without a separate power member.

The seating panel 3100 may extend upward from both side surfaces of the base panel 3200.

The base module 3000 may be coupled with the rail 3300 that is coupled to an inner surface of the seating panel 3100 and withdraws the withdrawal portion 1100.

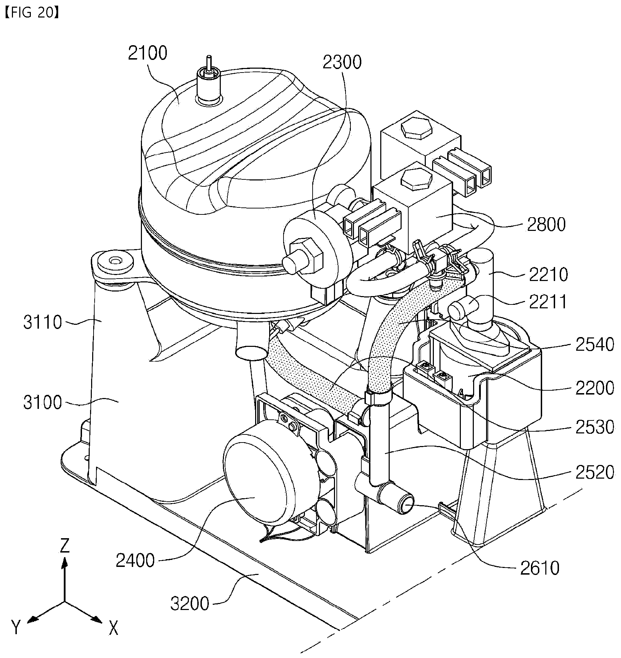

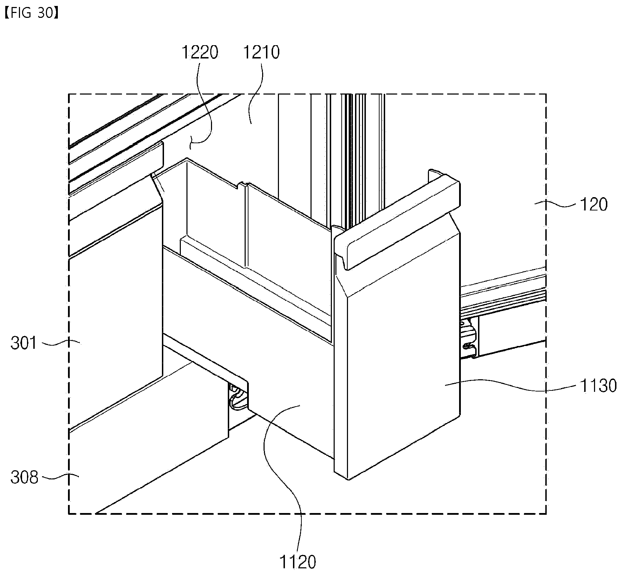

FIG. 20 shows a water supply and drainage structure of the heating module 2000. -FIG. 30

For the steam generator 2100 to generate steam and deliver steam to the steam iron 1300, water needs to be supplied.

To this end, the steam generator 2100 may include the high-pressure pump 2200 that is connected to the water source and supplies water to the steam generator 2100.

The high-pressure pump 2200 may be in communication with the water supply tank 301 and receive water from the water supply tank 301.

In one example, the high-pressure pump 2200 may receive water from a water source different from the water supply tank and supply water to the steam generator 2100.

In one example, the high-pressure pump 2200 may not simply supply water to the steam generator 2100, but may supply water while increasing the pressure inside the steam generator 2100. As a result, water may be heated while the pressure inside the steam generator 2100 is higher than an atmospheric pressure, thereby generating high-pressure steam.

The steam generator 2100 may be equipped with a pressure sensor 2300 that measures an internal pressure, and the connector 2800 for supplying power to the pressure sensor 2300 and the steam generator 2100 may be coupled to the steam generator 2100.

The pressure sensor 2300 may induce the high-pressure pump 2200 to be operated more when the pressure of the steam generator 2100 is lower than a reference pressure, and may induce water or steam inside the steam generator 2100 to be discharged when the pressure of the steam generator 2100 is higher than the reference pressure.

For example, the reference pressure may be set to be 2 bar to 3 bar higher than the atmospheric pressure.

The steam generator 2100 may be seated on the base module 3000 and fixed using a fastening member or the like, and the high-pressure pump 2200 may also be seated on the base module 3000.

The heating module 2000 may include a pump connection pipe 2540 that connects the high-pressure pump 2200 with the steam generator 2100. The pump connection pipe 2540 may be made of a flexible material like a rubber hose or the like and may guide high-pressure water into the steam generator 2100.

The high-pressure pump 2200 may include a pump discharge pipe 2210 that receives and discharges water, and a pump supply pipe 2211 that extends from the pump discharge pipe 2210 and supplies water to the pump connection pipe 2540.

The pump supply pipe 2211 may be branched from the pump discharge pipe 2210 so as to effectively transfer water of various pressures or high-pressure water, and the pump connection pipe 2540 may also be formed in a shape of a single pipe at one end, but may be formed as a plurality of branch pipes at the other end so as to be connected to the pump supply pipe 2211.

The water supply structure 2500 may include a water supply connection pipe 2510 that supplies water from the water source to the steam generator 2100 or the high-pressure pump 2200, and a branch supply pipe 2530 that supplies the water from the high-pressure pump 2200 to the steam generator 2100.

In one example, a drainage apparatus 2400 that discharges water inside the steam generator 2100 when the steam generator 2100 stops operating or when steam generated from the steam generator 2100 condenses to generate water may be included.

The drainage apparatus 2400 may be equipped as a pump or as a simple valve. The drainage apparatus 2400 may be disposed under the steam generator 2100 and receive water from the steam generator 2100.

The drainage structure 2600 may discharge water condensed in the steam generator 2100 or collected in the drainage apparatus 2400.

The drainage structure 2600 may include a drainage connection pipe 2610 extending from the drainage apparatus 2400, and an inflow pipe 2620 coupled to the drainage connection pipe 2610 to guide water discharged from the drainage apparatus 2400 to the drainage tank 302.

The drainage apparatus 2400 may be connected to both the water supply structure 2500 and the drainage structure 2600. As a result, the structure of supplying or discharging water to or from the steam generator 2100 is unified, simplifying a flow channel and saving space.

In this case, water supplied to an apparatus connection pipe 2520 may be delivered to the drainage apparatus 2400, and the drainage connection pipe 2610 may be in communication with the apparatus connection pipe 2520.

The drainage apparatus 2400 may be controlled to selectively open at least one of the drainage connection pipe 2610 and the apparatus connection pipe 2520. When the drainage apparatus 2400 closes at least one of the drainage connection pipe 2610 and the apparatus connection pipe 2520, water supplied from the pump connection pipe 2540 may be supplied to the steam generator 2100 through the apparatus connection pipe 2520.

When the drainage apparatus 2400 opens both the drainage connection pipe 2610 and the apparatus connection pipe 2520, water collected or condensed in the steam generator 2100 may be completely discharged via the drainage connection pipe 2610. Accordingly, the apparatus connection pipe 2520 may serve as both a flow channel for supplying water to the steam generator 2100 and a flow channel for discharging water from the steam generator 2100.

The drainage apparatus 2400 may be controlled by an independent controller to be described below.

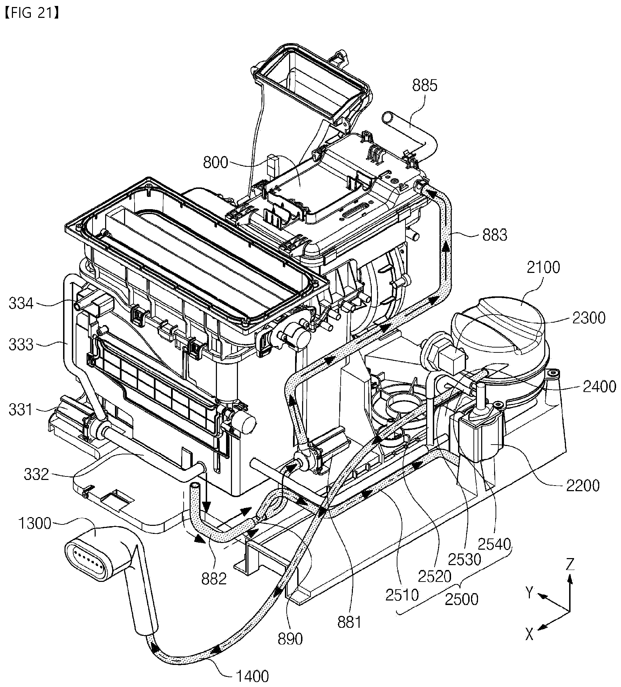

FIG. 21 shows a structure in which the water supply structure and the water supply are in communication with each other.

The ironing module S may be installed independently of and separately from the structure of the machine room 300, but may be in communication with the water supply 880 disposed in the machine room 300.

As a result, the ironing module S may receive water from the water supply tank 301. Accordingly, the ironing module S being connected to a separate water source or placing an additional water supply tank may be omitted. In addition, the clothing treatment apparatus may supply steam to both the inside of the inner casing 200 and the steam iron 1300 via the single water supply tank 301.

In one example, when the steam supply portion 800 is operated, the steam generator 2100 is controlled not to be operated, and when the steam generator 2100 is operated, the steam supply portion 800 is controlled not to be operated.

To this end, the clothing treatment apparatus of the present disclosure may further include a switching valve 890 that selectively supplies water contained in the water supply tank 301 to the steam supply portion 800 and the steam generator 2100.

The switching valve 890 may be disposed between the water supply pump 881 and the supply pipe 882 and may be controlled by a main controller 700.

The switching valve 890 may be equipped as a three-way valve.

When at least one of the closing of the door and the operation of the refreshing module T occurs, the switching valve 890 may be controlled to allow the water supply tank 301 and the water supply pump 881 to be in communication with each other and to block the communication between the water supply tank 301 and the water supply structure 2500.

When at least one of the opening of the door and termination of the operation of the refreshing module T occurs, the switching valve 890 may be controlled to block the communication between the water supply tank 301 and the water supply pump 881 and to allow the water supply tank 301 and the water supply structure 2500 to be in communication with each other.

When the switching valve 890 allows the water supply tank 301 and the water supply structure 2500 to be in communication with each other, the water supply connection pipe 2510 may receive water from the water supply tank 301 and transfer water to the high-pressure pump 2200.

The water supply connection pipe 2510 may have one end connected to the water supply 2501 and the other end connected to the high-pressure pump 2200.

In one example, the switching valve 890 may be equipped as a simple T-shaped pipe instead of the valve that changes the flow channel. In this case, when the water supply pump 881 is not operated and only the high-pressure pump 2200 is operated, water in the water supply tank 301 may be introduced into the high-pressure pump 2200 by a negative pressure. Water introduced into the high-pressure pump 2200 may be introduced into the steam generator 2100 via the pump connection pipe 2540 or the like.

In addition, when the water supply pump 881 is operated and the high-pressure pump 2200 is not operated, water in the water supply tank 301 may be supplied to the water supply pump 881 by the negative pressure, flow along the connection pipe 833, and be supplied to the steam supply portion 800.

Regardless of the configuration of the switching valve 890, when the steam generator 2100 is operated and steam is generated, steam may be transferred to the steam iron 1300 along the cable 1400 and sprayed to the outside of the steam iron 1300.

When the steam nozzle 900 is disposed separately, water supplied via the water supply pump 881 may be supplied to the steam nozzle 900 and then to the steam supply portion 800.

As a result, the clothing treatment apparatus of the present disclosure may supply water contained in the water supply tank 301 to both the steam supply portion 800 and the steam generator 2100. Therefore, installation of a separate water supply tank for the ironing module S may be omitted.

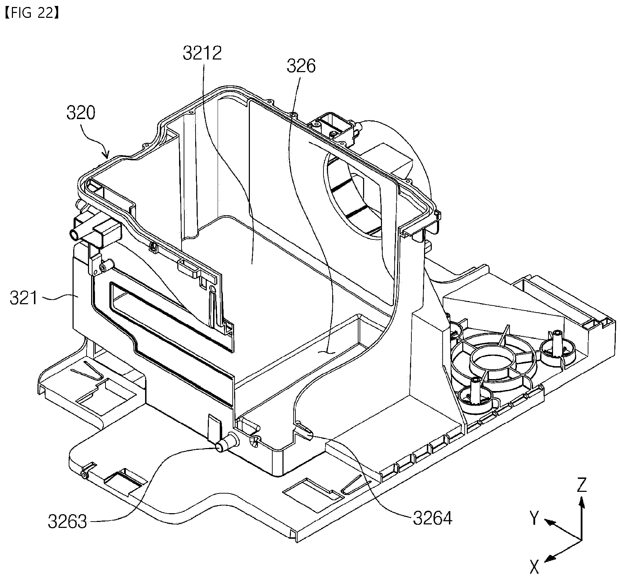

FIG. 22 shows a prerequisite structure in which the drainage structure and the drainage may be in communication with each other.

The circulation duct 320 may further include an inlet pipe 3264 that guides water discharged from the drainage structure 2600 to the reservoir 326.

A direction in which the inlet pipe 3264 is directed and a direction in which the guide pipe 3263 is directed may be different from each other.

For example, the guide pipe 3263 may be formed on the front surface of the circulation duct 320, and the inlet pipe 3264 may be formed on a left side surface or a right side surface of the circulation duct 320.

The inlet pipe 3264 may be disposed in an area corresponding to a side surface of the reservoir 326 in the side surface of the circulation duct 320.

The inlet pipe 3264 may allow the inside of the reservoir 326 and the outside of the circulation duct 320 to be in communication with each other, and may protrude outward from the side surface of the circulation duct 320.

The inlet pipe 3264 may be installed at a vertical level higher than that of a bottom surface of the reservoir 326 or the guide pipe 3263.

When the drainage structure 2600 discharges water to the inlet pipe 3264, water may be collected in the reservoir 326 and discharged via the guide pipe 3263 to be in communication with the drainage 330.

Therefore, a separate drainage tank for the ironing module S may be omitted.

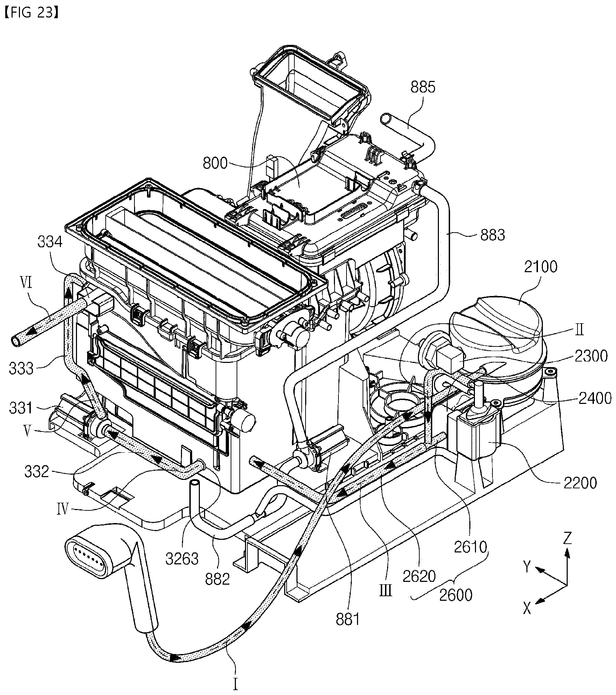

FIG. 23 shows a structure in which the drainage structure and the drainage are in communication with each other.

The ironing module S may be installed independently of and separately from the structure of the machine room 300, but may be in communication with the drainage 330 of the refreshing module T via the circulation duct 320.

As a result, the ironing module S may discharge residual water into the drainage tank 302. Therefore, a structure in which the ironing module S includes an additional drainage tank 302 or is connected to a separate sewer may be omitted.

The clothing treatment apparatus of the present disclosure may collect both residual water collected in the circulation duct 320 and residual water collected in the ironing module S with the single drainage tank 302. The user may discharge both water collected in the circulation duct 320 and the ironing module S by dumping water in the drainage tank 302.

The drainage structure 2600 may be in communication with the drainage 330 via the circulation duct 320.

The drainage structure 2600 may include the drainage connection pipe 2610 connected to the drainage apparatus 2400, and the inflow pipe 2620 connected to the drainage connection pipe 2610 and in communication with the drainage 330. The inflow pipe 2620 may be connected to the inlet pipe 3264.

Water condensed in the steam generator 2100 and residual water may be collected in the drainage apparatus 2400 (direction II). Water condensed in the steam iron 1300 may flow along the cable 1400 and be collected in the drainage apparatus 2400 via the steam generator 2100 (direction I). Water collected in the drainage apparatus 2400 may be connected to the inlet pipe 3264 along the inflow pipe 2620. Water may be collected in the drainage tank 302 (direction III). That is, when the drainage pump 331 is operated, water collected in the drainage apparatus 2400 may be collected in the drainage tank 302 by the negative pressure.

The inflow pipe 2620 may be in communication with the inside of the circulation duct 320.

As a result, all of water collected in the drainage apparatus 2400 may be collected inside the circulation duct 320 and then flow to the drainage pump 331 via the guide pipe 3263. Even when an amount of water collected in the drainage apparatus 2400 is large or an amount of water discharged via the drainage apparatus 2400 is large, water may be collected inside the circulation duct 320, thereby preventing water from overflowing out of the machine room 300.

In one example, the drainage apparatus 2400 may be mounted on the base module 3000 and positioned at a vertical level higher than that of a distal end of the inflow pipe 2620. Accordingly, even when the drainage apparatus 2400 does not generate any special power, water collected in the drainage apparatus 2400 may be collected in the reservoir 326 along the inflow pipe 2620.

When the drainage pump 331 is operated, water collected in the reservoir 326 may be stored in the drainage tank 302.

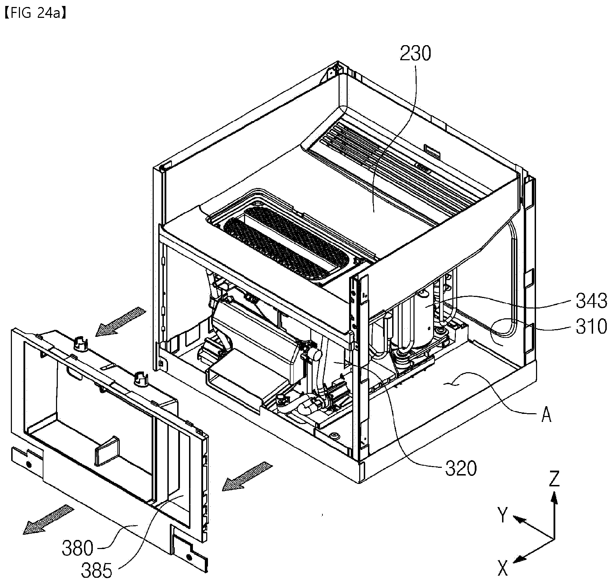

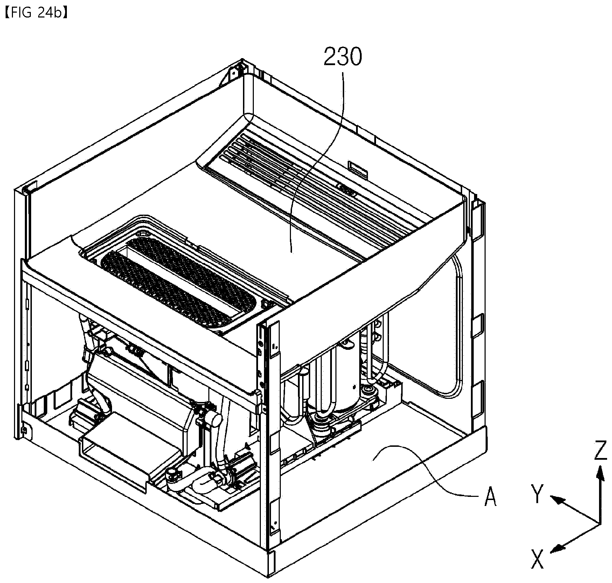

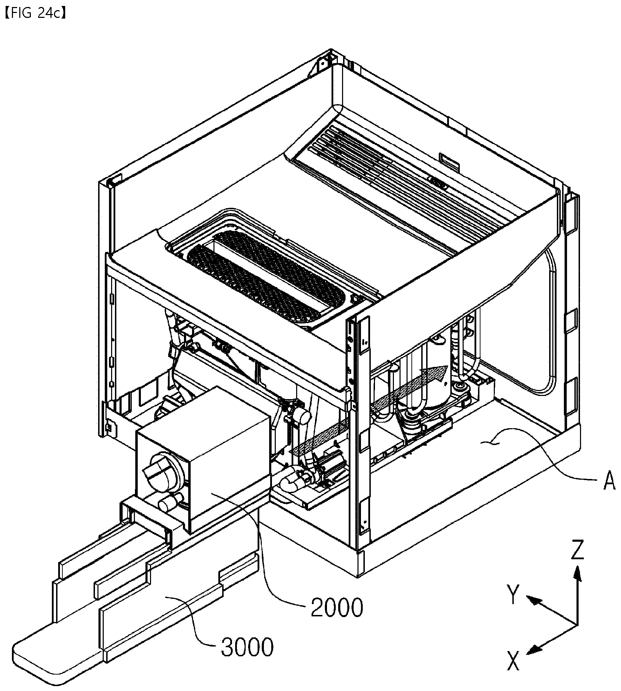

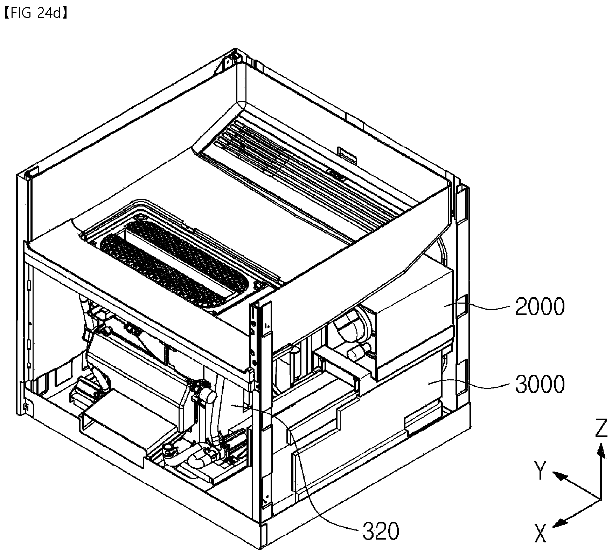

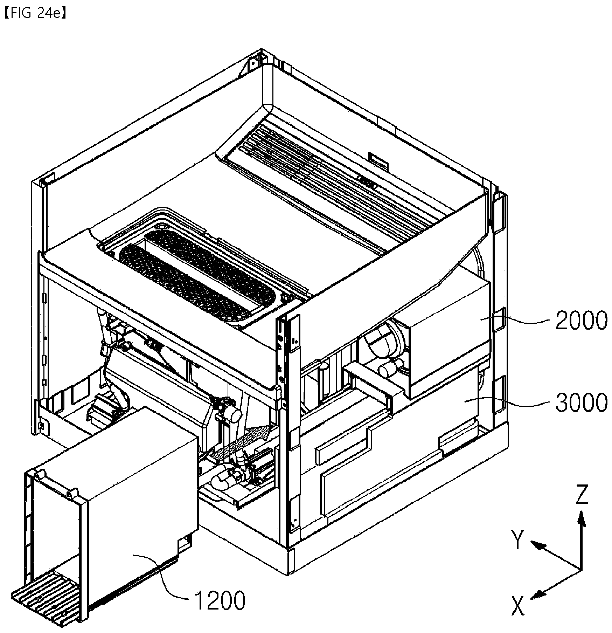

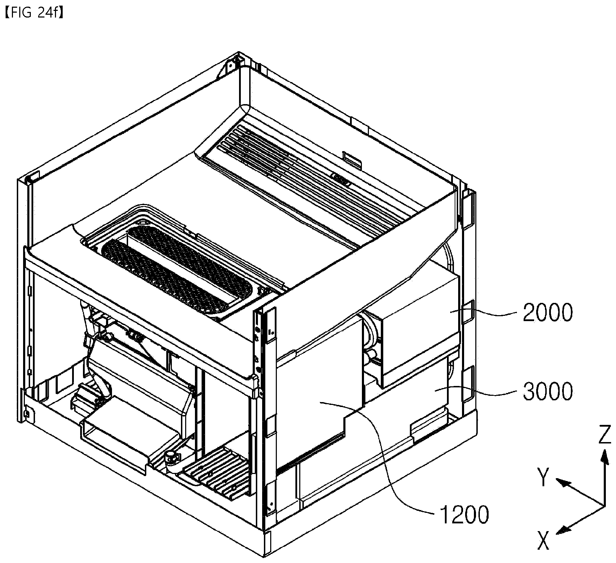

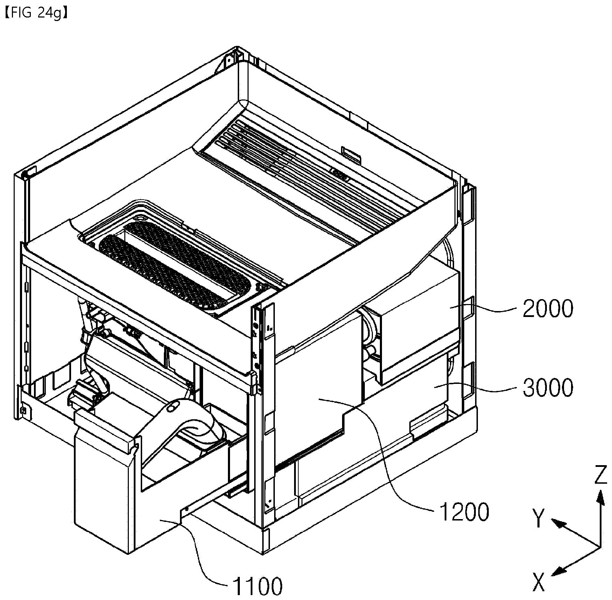

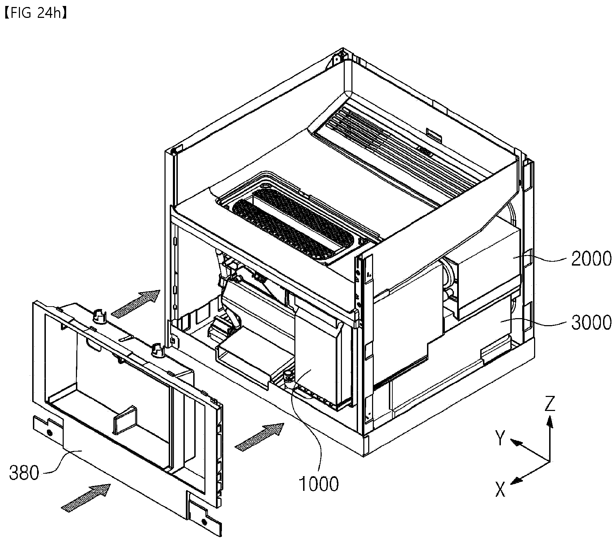

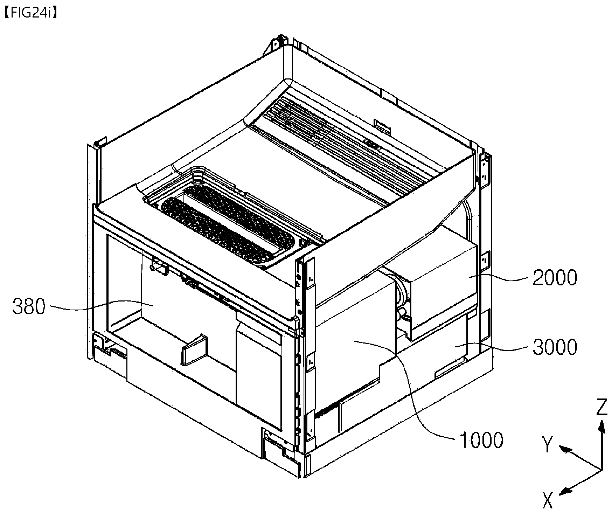

FIG. 24 shows an embodiment in which the iron module S of the present disclosure is installed in a machine room.

The iron module S of the clothing treatment apparatus of the present disclosure may be additionally installed in the cabinet 100 in which the machine room 300 is already equipped. That is, in a state in which the circulation duct 320, the heat supply portion 340, the steam supply portion 800, the water supply 880, the drainage 330, the blower 350, and the like are all installed in the machine room 300, the iron module S may be additionally installed.

In addition, in a state in which the clothing treatment apparatus is sold without the iron module S installed inside, the user may separately purchase the iron module S and install the iron module S in the machine room 300.

Therefore, the clothing treatment apparatus in which the iron module S is installed and the clothing treatment apparatus in which an iron module I is omitted may be independently produced, and only the iron module S may be manufactured separately and mounted in the clothing treatment apparatus in which the iron module S is omitted.

Therefore, a user's choice may be guaranteed, and designing and manufacturing of a separate clothing treatment apparatus model to manufacture the clothing treatment apparatus equipped with the iron module S may be prevented.

Referring to (a) in FIG. 24, it may be a state in which a space S in which the iron module S is installed is defined inside the machine room 300, and the iron module S is omitted.

The space S may be defined on one side of the circulation duct 320. The space S may be defined close to the water supply tank 301.

The space S may be defined to be in communication with front and rear areas outside the circulation duct 320.

A width of the circulation duct 320 or the base 310 may be smaller than a width of the entire machine room 300. The width of the machine room 300 may be greater than the widths of the circulation duct 320 and the base 310.

A width of the space S may correspond to a width of the iron module S.

The detachment portion 380 may be in a state of shielding a front portion of the machine room 300.

The detachment portion 380 may further include a through-hole 385 through which the iron module S may pass or through which the steam iron 1300 may be inserted and withdrawn.

The through-hole 385 may be defined on a side of the water supply tank 301.

Referring to (b) in FIG. 24, to install the iron module S, the detachment portion 380 may be separated forward of the machine room 300. As a result, the space S may be exposed forward of the machine room 300.

A rear portion of the machine room 300 may be maintained in a state of being shielded by a rear panel of the cabinet 100. In addition, both side surfaces of the machine room 300 may be maintained in the shielded state, and as shown, only a side panel of the cabinet 100 facing the space S may be separated.

The machine room 300 may include a bottom panel forming a bottom surface of the space S. The bottom panel may fix the base 310 to the cabinet 100.

Referring to (c) in FIG. 24, the heating module 2000 may be mounted on the base module 3000, outside the machine room 300. Thereafter, the base module 3000 and the heating module 2000 may be simultaneously introduced into the front portion of the machine room 300 and installed in the space S.

The base module 3000 may be detachably fixed to the bottom panel or a frame.

The base module 3000 may be introduced into the front portion of the machine room 300 and seated in the space S, and then the heating module 2000 may be introduced into the front portion of the machine room 300 and seated on the base module 3000.

Referring to (d) in FIG. 24, the base module 3000 and the heating module 2000 may be seated in the space S. In this process, the heating module 2000 may be in communication with the water supply 880 and the drainage 330. In addition, the heating module 2000 may be connected to the main controller 700 or a power supply.

Referring to (e) in FIG. 24, the storage module 1000 may be installed in the machine room 300. The storage module 1000 may be assembled entirely outside the machine room 300 and then introduced into the machine room 300 as a whole.

However, as shown, the storage module 1000 may be introduced into the machine room 300 in stages for convenience.

The housing 1200 may be introduced into the front portion of the machine room 300 and mounted on the base module 3000.

Referring to (f) in FIG. 24, the housing 1200 may be mounted on the base module 3000.

Referring to (g) in FIG. 24, the withdrawal portion 1100 may be inserted into the housing 1200. The steam iron 1300 may be mounted on the withdrawal portion 1100, and the steam iron 1300 and the withdrawal portion 1100 may be inserted into the housing 1200 at the same time. The cable 1400 connected to the steam iron 1300 may be accommodated inside the withdrawal portion 1100, and only a distal end of the cable 1400 may be exposed so as to be coupled to the heating module 2000.

Referring to (h) in FIG. 24, when the storage module 1000 is installed in the machine room 300, the front portion of the machine room 300 may be shielded via the detachment portion 380. As a result, the inside of the machine room 300 may be blocked from being exposed forward.

Referring to (i) in FIG. 24, when the detachment portion 380 shields the front portion of the machine room 300, the iron module S may shield the through-hole defined in the detachment portion 380 and may be exposed forward of the detachment portion 380.

In addition, the water supply tank 301 and the drainage tank 302 may be sequentially seated on the detachment portion 380 to improve overall aesthetics.

The iron module S of the clothing treatment apparatus of the present disclosure may be independently installed in the machine room 300 in the modular manner, while the respective components may be installed in the machine room 300 in the modular manner in stages. As a result, convenience of installation may be increased.

In one example, the iron module S may be assembled as a whole outside the machine room 300 and then installed in the space S by being introduced into the machine room 300 as a whole.

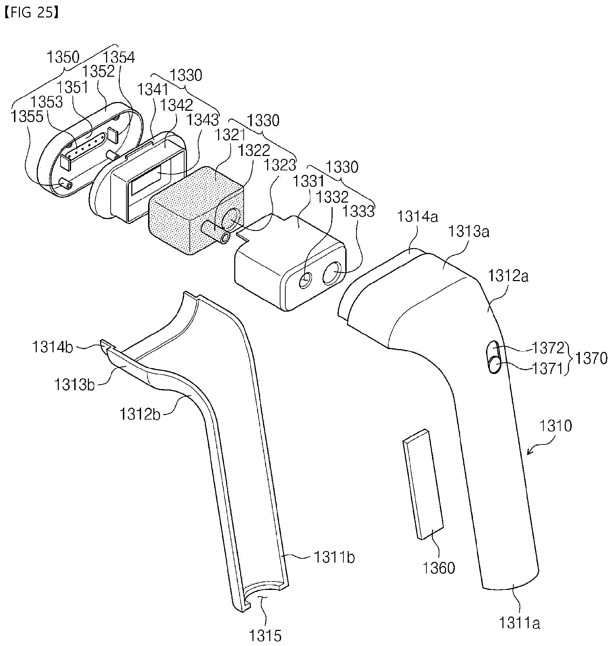

FIG. 25 shows a structure of the steam iron.

The steam iron 1300 of the present disclosure may include a head 1310 connected to the cable 1400 and provided to be gripped by the user.

The head 1310 may spray and supply at least one of water and steam to the surface of the laundry.

The head 1310 may include a handle 1311 that may support a weight of the steam iron 1300, and an expansion portion 1313 extended from an upper end of the handle 1311.

The head 1310 may have an installation space defined therein through which steam may pass and in which various components may be installed, and at least one of steam and heat may be discharged via a distal end of the head 1310.

The head 1310 may have a length greater than a width or a thickness, thereby preventing a user's body from being damaged by steam and heat discharged from the distal end of the head 1310.

The head 1310 may include the handle 1311 formed in a pipe shape or the like to provide an area that a user's hand may grip, a bent portion 1312 bent from the handle 1311, and the expansion portion 1313 that extends from the bent portion 1312 to a distal end.

The handle 1311 may be formed in a cylindrical shape to accommodate the cable 1400 therein and may have a length greater than a diameter. As a result, a space that the user may grip may be secured.

The bent portion 1312 may be bent within 90 degrees at a distal end of the handle 1311. Because of the bent portion 1312, at least one of steam and heat flowing along the handle 1311 may easily encounter the laundry.

The expansion portion 1313 may have the greatest cross-sectional area compared to the bent portion 1312 and the handle 1311. As a result, at least one of steam and heat may be induced to be supplied to a wide area.

A space in which a heating apparatus or the like that increases a temperature of supplied steam and heat may be accommodated may be defined in the expansion portion 1313.

The head 1310 may be formed in two pieces to facilitate installation and accommodation of internal components thereof.

For example, the head 1310 may include a lower head 1310b that may form a bottom surface and a portion of both side surfaces of the head 1310, and an upper head 1310a that may form a top surface and a remaining portion of both side surfaces of the head 1310.

The head 1310 may further include a converging portion 1314 that extends further from the expansion portion 1313 to the distal end, but has a smaller cross-sectional area than the expansion portion 1313. The converging portion 1314 may prevent the internal components of the gripping portion from being arbitrarily discharged to the outside.

The converging portion 1314 may have a hollow defined therein, thereby allowing at least one of steam and heat transferred up to the expansion portion 1313 to flow to the outside of the steam iron 1300.

The steam iron 1300 may include a heater base 1320 that may be accommodated in the head 1310 and may heat water and steam supplied from the steam generator 2100.

The heater base 1320 may be heated by receiving power from an independent controller 4000, which will be described below. As a result, steam transmitted via the cable 1400 may be heated again and be prevented from being condensed, and wrinkles on the surface of the laundry that comes into contact with the steam iron 1300 may be removed.

The heater base 1320 may be formed in a casing shape that accommodates steam or water supplied from the steam generator 2100 therein, and may additionally heat the steam and water.

As a result, even when the steam is condensed into water, water may be heated again and sprayed as steam in the heater base 1320, and even before steam is condensed into water, steam may be reheated and prevented from being condensed into water.

The heater base 1320 may be supplied with power and generate heat. To this end, the head 1310 of the present disclosure may further include a steamer panel 1360 that may control on/off of the heater base 1320. The steamer panel 1360 may be equipped as a PCB panel, and may be controlled such that the heater base 1320 is heated when a temperature of the heater base 1320 falls to a specific temperature or below.

The heater base 1320 may include a heater flow hole 1323 through which steam supplied from the cable 1400 flows, and a heater receiving pipe 1322 through which a power line moving along the cable 1400 moves.

The heater flow hole 1323 and the heater receiving pipe 1322 may be defined in and disposed on a rear surface of the heater base 1320, and the heater base 1320 may be made of a metal material having a low specific heat. The heater base 1320 may have a discharge surface at a front side that discharges steam and heat to the outside.

The steam iron 1300 of the present disclosure may include an iron cover 1350 that discharges at least one of heat and steam discharged from the heater base 1320 and prevents the heater base 1320 from being exposed from the head 1310.

The iron cover 1350 may form a distal end of the steam iron 1300.

The iron cover 1350 may include a coupling surface 1352 coupled to a distal end of the head 1310, an exposed surface 1351 extending from the coupling surface 1352 to form a distal end surface of the steam iron 1300, and a spray hole 1353 extending through the exposed surface 1351 to spray steam to the outside. In addition, the iron cover 1350 may include a fixing fastener 1355 that may be coupled and fixed to the head 1310 or the internal components, and may include guide ribs 1354 respectively disposed on both sides of the injection hole 1353 to guide the flow of the steam.

The exposed surface 1351 may be disposed flatly so as to be in contact with the surface of the laundry, and may transfer steam and heat generated from the heater base 1320 to the surface of the laundry.

The steam iron 1300 of the present disclosure may further include a fixing bracket 1340 on the head 1310 that fixes the heater base 1320 to the distal end of the head 1310.

The fixing bracket 1340 may include an accommodating peripheral surface 1342 that accommodates the front surface of the heater base 1320 therein, a transfer hole 1343 that is defined inside the accommodating peripheral surface and transfers at least one of steam and heat discharged from the heater base 1320 to the iron cover 1350, and an expanding body 1341 that is expanded from the accommodating peripheral surface 1342 and comes into contact with the coupling surface 1352.

The steam iron 1300 of the present disclosure may include an insulating accommodating portion 1330 that accommodates the heater base 1320 therein and insulates the heater base 1320.

The insulating accommodating portion 1330 may include an accommodating body 1331 that accommodates the heater base 1320 therein, a passage hole 1333 that extends through a rear portion of the accommodating body 1331 and allows steam supplied from the cable 1400 to pass therethrough, and a power hole 1332 through which the power line extended from the cable 1400 passes.

The accommodating body 1331 may accommodate therein all surfaces except the front surface of the heater base 1320, and may be made of an insulating material or a material having a much higher specific heat than the heater base 1320. As a result, the heater base 1320 may be prevented from being cooled, thereby preventing steam from being condensed or thermal efficiency of the steam iron 1300 from being reduced.

The head 1310 may include an operating input unit 1370 that operates the heater base 1320 or operates the steam iron 1300 itself.

The operating input unit 1370 may include a decision input unit 1371 that moves in a vertical direction or in a left and right direction to determine on/off of the heater base 1320 or the like, and a guide groove 1372 that guides the movement of the decision input unit 1371.

When the decision input unit 1371 is disposed at an 'on' location, the heater base 1320 may be operated, or at least one of steam and heat may be controlled to pass through the heater base 1320.

In addition, when the decision input unit 1371 is disposed at an 'off' location, the operation of the heater base 1320 may be stopped, or both steam and heat may be controlled not to pass through the heater base 1320. Accordingly, even when the heating module 2000 is operated, arbitrary spraying of at least one of heat and steam from the steam iron 1300 may be prevented.

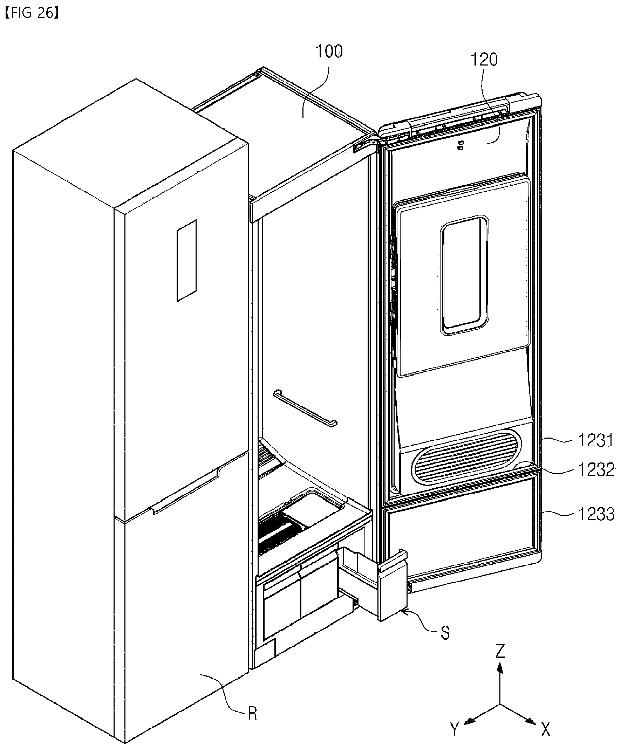

FIG. 26 shows characteristics of a direction in which the steam iron is withdrawn.

The iron module S may be provided to be exposed forward of the machine room 300, and may be blocked from being exposed via top/side/rear surfaces of the machine room 300.

The iron module S may be exposed to the outside only when the door 120 is opened, and may be completely blocked from being exposed to the outside of the cabinet 100 when the door 120 is closed.

In the iron module S, the steam iron 1300 may be withdrawn forward of the machine room 300. That is, the withdrawal portion 1100 may be withdrawn or rotated forward of the machine room 300 to open the open surface of the housing, and may expose the steam iron 1300 forward of the machine room 300.

As a result, when the door 120 opens the opening, the steam iron 1300 may be withdrawn forward of the machine room 300.

In addition, even when a separate home appliance R or a wall is disposed on each of both side surfaces of the clothing treatment apparatus, when only a space in which the door 120 may be opened is secured, the steam iron 1300 may be withdrawn from and accommodated in the machine room 300 without any restrictions.

Therefore, the clothing treatment apparatus of the present disclosure may maintain a freedom of installation without being subject to space restrictions even when the iron module S is installed.

In addition, the iron module S of the present disclosure may be exposed only forward of the machine room 300 and not into the inner casing 200. That is, when the steam iron 1300 and the withdrawal portion 1100 are accommodated in the machine room 300, the inner casing 200 and the iron module S may not face each other or not be in communication with each other.

As a result, the foreign substances and moisture that may exist in the inner casing 200 may be prevented from being exposed to the iron module S. In addition, air inside the inner casing 200 may be prevented from being introduced into the iron module S.

Furthermore, even when the machine room 300 has the through-hole through which the iron module S may be installed or the steam iron 1300 may be inserted and withdrawn, the moisture, the foreign substances, and air that may remain inside the inner casing 200 may be prevented from being introduced into the through-hole.

Therefore, durability of not only the iron module S but also the entire machine room 300 may be guaranteed.

In one example, the sealing member 123 attached to the inner surface of the door 120 may include a partition member 1232 that partitions a lower end of a front surface of the opening 210 or the inner casing 200 from the top surface of the machine room 300.

The partition member 1232 may be in contact with a front side of the lower end of an outer surface of the opening 210, and may have a length equal to or greater than a length of the lower end of the opening 210.

The partition member 1232 may be formed in a rod shape, and may be made of an elastic member to pressurize and seal the lower end of the opening 210.

Fluid contained inside the inner casing 200 may be prevented from flowing to the machine room 300 by being blocked by the partition member 1232.

In one example, the sealing member 123 may further include an airtight member 1231 that extends upward from both ends of the partition member 1232 to seal both side surfaces and the top surface of the opening 210.

In one example, the sealing member 123 may further include a blocking member 1233 that extends downward from both ends of the partition member 1232 to seal an outer peripheral surface of the machine room 300.

As a result, fluid disposed in a front portion of the machine room 300 may be prevented from leaking out of the door 120, and at the same time, fluid outside the door 120 may be prevented from being introduced into the front portion of the machine room 300.

The partition member 1232, the airtight member 1231, and the blocking member 1233 may all be integrally formed or may all be formed as separate components.

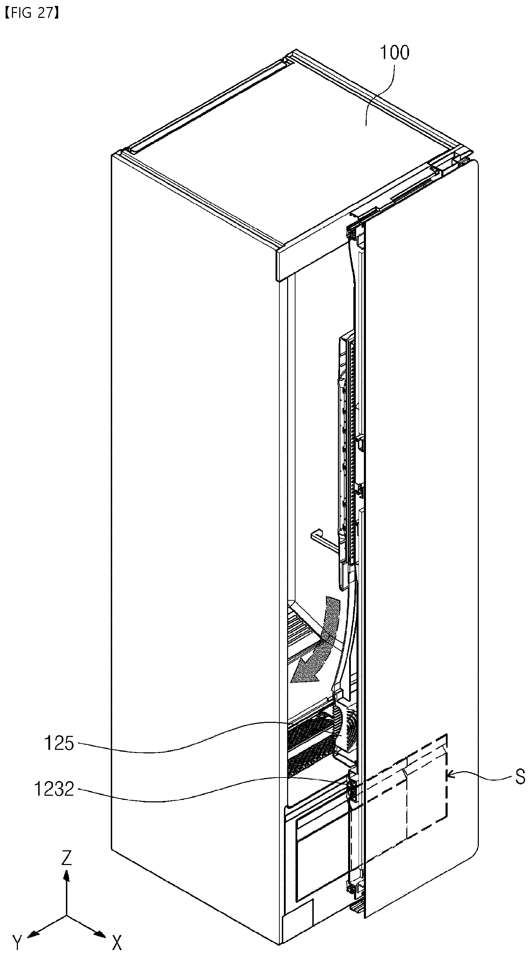

FIG. 27 shows a structure in which the steam iron is protected from foreign substances.

The door 120 may be provided such that the sealing member 123 is in close contact with a periphery of the opening 210. Particularly, the partition member 1232 may be attached in a width direction to a location between an exposed surface of the bottom surface 230 of the inner casing 200 and the top surface of the machine room 300.

The machine room 300 may be disposed below the partition member 1232. In addition, a periphery of a front surface of the machine room 300 may also be sealed by the blocking member 1233.

As a result, when the door 120 closes the opening 210, the accommodating space of the inner casing 200 may be completely sealed. Therefore, hot air and steam supplied to the inner casing 200 may be finally discharged via the inflow hole 232 while circulating in the accommodating space 220 without passing through the opening 210 by the partition member 1232 and the airtight member 1231.

In addition, the foreign substances removed from the laundry hung in the inner casing 200 may also be finally discharged via the inflow hole 232 while circulating in the accommodating space 220 without passing through the opening 210.

In addition, the iron module S may be completely fluidically partitioned and separated from the inner casing 200 by the partition member 1232 attached to the door 120. Therefore, the iron module S may be prevented from coming into contact with air, moisture, and the foreign substances existing in the inner casing 200 by the partition member 1232. Cleanliness of the iron module S may be maintained, the iron module S may be prevented from being damaged by heat, or corrosion and electrical accidents of the iron module S caused by moisture may be prevented.

In one example, the door 120 includes the protruding portion 125 protruding from an inner surface thereof, and the protruding portion 125 is disposed close to a bottom surface of the opening 210. That is, the protruding portion 125 protrudes from the door inner surface 121 and is at least partially inserted into the opening 210, thereby facing the bottom surface of the opening 210 and the bottom surface 230 of the inner casing 200.

Therefore, air, moisture, the foreign substances, and the like circulating or flowing inside the inner casing 200 may be obstructed by the protruding portion 125 from flowing to the bottom surface of the opening 210, and may be guided to be discharged via the inflow hole 232.

As a result, air, moisture, the foreign substances, and the like circulating or flowing inside the inner casing 200 are primarily restricted by the protruding portion 125, and secondarily blocked by the partition member 1232 so as not to leak out of the opening 210. Accordingly, the iron module S including the steam iron 1300, which may be exposed forward of the machine room 300, may be protected.

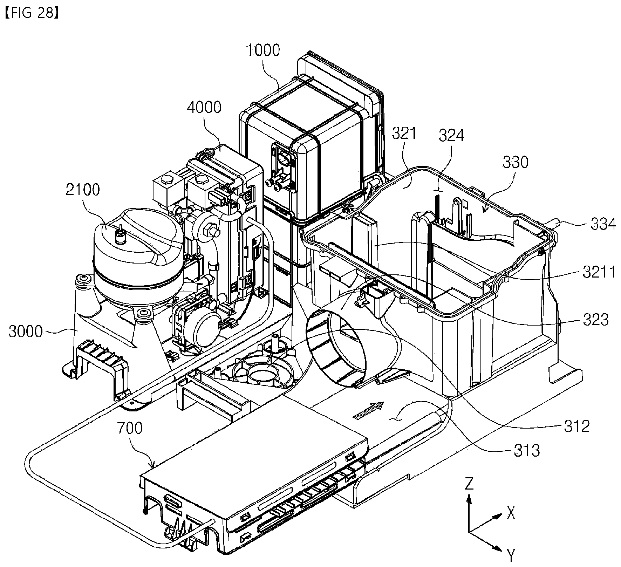

FIG. 28 shows a structure and an arrangement of an iron module and a controller.

The main controller 700 may be connected to the external power source and supplied with the power.

The main controller 700 may not only supply the power to the refreshing module T, such as the heat supply portion 340, the steam supply portion 800, the water supply 880, the drainage 330, and the blower 350, but also supply the power to all of the electrical components of the clothing treatment apparatus 1.

The main controller 700 may also supply the power to the iron module S.

However, the iron module S should supply the power to the heating module 2000, which heats water to generate steam, and should also supply the power for boiling water to the heater base 1320 equipped in the steam iron 1300. In addition, the iron module S should determine whether to operate the heating module 2000, the steam iron 1300, the water supply structure 2500, and the drainage apparatus 2400, and control them.

In this case, when the main controller 700 is provided to supply the power to the iron module S or control the iron module S, a size of the main controller 700 should be greater than an area size of the circulation duct 320.

Accordingly, the iron module S may further include the independent controller 4000 that supplies the power to at least one of the heating module 2000 including the steam generator 2100, the steam iron 1300 including the heater base 1320, the water supply structure 2500, and the drainage apparatus 2400.

In addition, when the independent controller 4000 is provided to not only supply sufficient power to the heating module 2000 including the steam generator 2100, but also determine whether to supply the power to the heating module 2000 and control the heating module 2000, a volume of the independent controller 4000 may become similar to that of the steam iron 1300.

In addition, when the independent controller 4000 is provided to supply the power to heat the heater base 1320 mounted on the steamer module 1300 and control the same without having to consider the heating module 2000, the independent controller 4000 should have an area size similar to that of the head of the steam iron 1300.

Therefore, when the independent controller 4000 is disposed inside the steam iron 1300, the steam iron 1300 may become too large for the user to grip, and a weight of the steam iron 1300 may also make it impossible for the user to grip the steam iron 1300.

Therefore, the iron module S of the present disclosure may not install the independent controller 4000 in the steam iron 1300, but install the independent controller 4000 inside the machine room 300.

As a result, the steamer panel 1360 of the steam iron 1300 may be simply provided to receive the power from the independent controller 4000, then simply transmit the power to the heater base 1320, then receive a signal for controlling the heater base 1320 from the independent controller 4000, and then only determine ON/OFF of the heater base 1320. As a result, the steamer panel 1360 may be miniaturized.

The iron module S may further include a power line L2 accommodated in the cable 1400 and electrically connected to at least one of the independent controller 4000, the steamer panel 1360, and the heater base 1320.

The independent controller 4000 may supply the power to at least one of the steamer panel 1360 and the heater base 1320 via the power line L2, and may determine a control signal for controlling the heater base 1320 and transmit the signal to the steamer panel 1360.

The independent controller 4000 may be mounted on the base module 3000 and may be disposed close to the heating module 2000 to minimize electrical resistance.

Because the iron module S is disposed on either the left or right side of the refreshing module T, the independent controller 4000 may also be disposed on either the left or right side of the refreshing module T.

In one example, the iron module S needs to be operated independently of the various refreshing modules T that perform a cycle of refreshing the laundry hung inside the inner casing 200.

In addition, the refreshing module T, such as the heat supply portion 340, the steam supply portion 800, the water supply 880, the drainage 330, and the blower 350, is controlled to operate when the door 120 closes the opening 210, and the iron module S is controlled to operate when the door 120 opens the opening 210.

Therefore, the independent controller 4000 may be equipped independently of the main controller 700 to control the electrical components of the iron module S.

The independent controller 4000 may not be directly connected to the external power source, but may be electrically connected to the main controller 700 to receive required power from the main controller 700. In this case, the main controller 700 may perform a role of the external power source for the iron module S.

Therefore, the clothing treatment apparatus of the present disclosure may further include a control line L1 that electrically connects the independent controller 4000 with the main controller 700.

The independent controller 4000 may receive various types of information from the main controller 700 via the control line L1. The independent controller 4000 may receive information on whether the door 120 has closed the opening 210, which may be recognized by the main controller 700, and information on whether the refreshing module T is operating, via the control line L 1.

The independent controller 4000 may receive the power from the main controller 700 via the control line L1. That is, the independent controller 4000 may receive the power via the control line L1 and supply a large amount of power to boil water in at least one of the heating module 2000 and the steam iron 1300.

The independent controller 4000 may be equipped to supply the power to at least one of the steam generator 2100, the high-pressure pump 2200, the drainage apparatus 2400, and the internal components of the steam iron 1300.

In summary, it is impossible for the independent controller 4000 to have a volume to be installed inside the steam iron 1300 to supply the power to or control at least one of the steam generator 2100, the high-pressure pump 2200, the drainage apparatus 2400, and the internal components of the steam iron 1300.

That is, to design the steam iron 1300 with a volume and a weight that allow the steam iron 1300 to be gripped by the user with hand, the steam iron 1300 should be disposed separately from the independent controller 4000.

Therefore, in the iron module S of the present disclosure, the independent controller 4000 is preferably disposed separately and spaced apart from the steam iron 1300.

The independent controller 4000 may be seated on and fixed to the base module 3000.

The independent controller 4000 may be disposed between the heating module 2000 and the storage module 1000. As a result, the power line L2 connecting the independent controller 4000 to the heating module 2000 and the steam iron 1300 may be minimized in length, and the independent controller 4000 may be prevented from being exposed to external impact or vibration.

The iron module S may be operated regardless of the control state of the refreshing module T because of the independent controller 4000.

However, the iron module S is provided to operate only when the door 120 is opened, and the heat supply portion 340 and the steam supply portion 800 should be controlled to operate on the premise that the door 120 is not opened. Therefore, the independent controller 4000 is provided to receive the power from the main controller 700 via the control line L1 and be in communication with the main controller 700 at the same time, so that the iron module S may be controlled by recognizing the opening state of the door 120 and the operating states of the heat supply portion 340 and the steam supply portion 800.

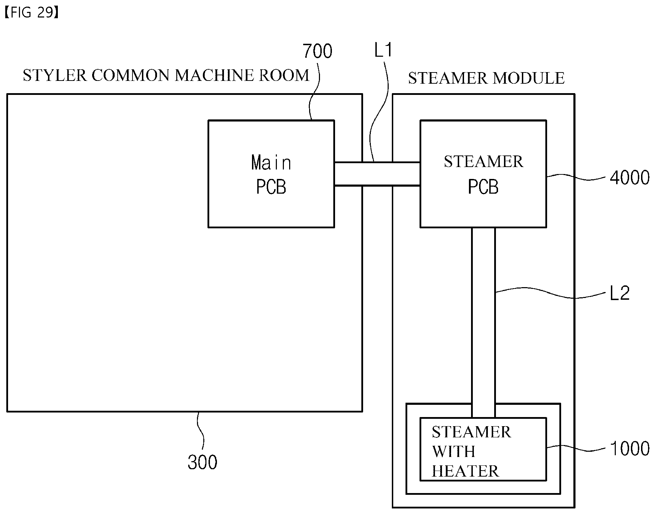

FIG. 29 is a diagram illustrating arrangement of the controllers.

The components seated on the base 310 of the machine room 300 may be defined as a common machine room 300, and the internal components of the machine room 300 may be controlled by the main controller 700.

In the iron module S, the independent controller 4000 may be separated from the steam iron 1300 and mounted on and fixed to the base module 3000, separated from the main controller 700.

The independent controller 4000 may be connected to the steam iron 1300 via the power line L2 and control the spraying of at least one of steam and heat from the steam iron 1300. For example, the independent controller 4000 may control the heater base 1320 and the like.

In addition, the main controller 700 may be connected to the independent controller 4000 via the control line L1 and in communication with the independent controller 4000. The independent controller 4000 may be equipped with one or more PCB panels, and may have an area size equal to or greater than an area size of the steam iron 1300.

FIG. 30 shows an embodiment in which the iron module is equipped in a drawer manner.

In the machine room 300, the iron module S may be disposed closer to the water supply tank 301 than to the drainage tank. As a result, water remaining in the water supply structure may be minimized, thereby saving water and preventing water from being polluted. In addition, because the drainage structure is able to be guided to the circulation duct 320 by gravity even when a length thereof is great, the drainage tank 302 may be relatively far from the iron module S without any issues.

The iron module S may be equipped with the support portion 1120 that is withdrawn forward on one side of the water supply tank 301, and the cover 1130 that extends in front of the support portion 1120 and blocks the internal components of the iron module S from being exposed to the outside.

The support portion 1120 may be formed in a casing shape that may accommodate the steam iron 1300 therein, and may have a vertical dimension smaller than that of the cover 1130, but may have a length in the front and rear direction greater than that that of the cover 1130.

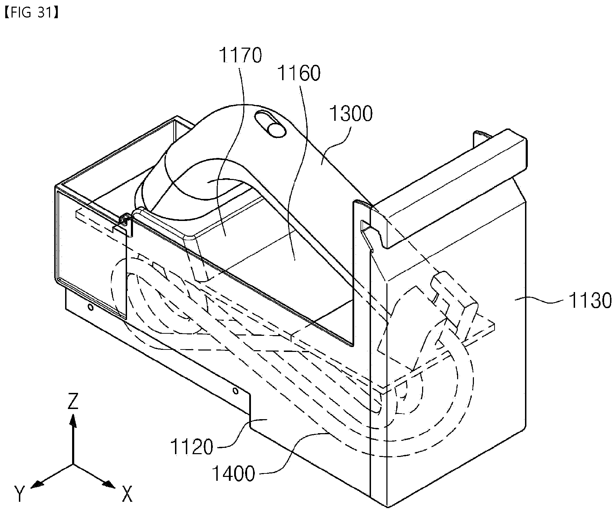

FIG. 31 shows an embodiment in which a steam iron is stored in the withdrawal portion.

The steam iron 1300 may be withdrawn out of the housing 1200 and may be brought close to a specific area of the laundry to remove the wrinkles. To this end, the cable 1400 connecting the steam iron 1300 with the heating module 2000 may have a length greater than that of the support portion 1120.

However, when the operation of the steam iron 1300 is stopped, the steam iron 1300 and the cable 1400 need to be stably accommodated in the housing 1200.

To this end, the withdrawal portion 1100 may include the support portion 1120 having a settling space that may accommodate the entire cable 1400 therein. Accordingly, because the entire cable 1400 is accommodated in the support portion 1120, even when the cable 1400 is long, the cable 1400 may be stably stored in the support portion 1120 and may not interfere with the closing of the door 120 or the like.

However, the support portion 1120 may expose a portion of the steam iron 1300. This is to facilitate the user's gripping of the steam iron 1300.

The withdrawal portion 1100 may further include a partition panel 1160 that may partition an internal space of the support portion 1120 and may support a lower portion of the steam iron 1300.

The partition panel 1160 may be rotatably coupled to both side surfaces at a rear portion of the support portion 1120 to partition the internal space of the support portion 1120 into upper and lower portions.

In the withdrawal portion 1100, an area for fixing the handle 1311 of the steam iron 1300 and an area for supporting the iron cover 1350 of the steam iron 1300 may be separated from each other.

The withdrawal portion 1100 may be detachable from the iron cover 1350, and may prevent the steam iron 1300 from moving arbitrarily when the steam iron 1300 is seated therein.

The withdrawal portion 1100 may further include a mounting panel 1170 that extends from one end of the partition panel 1160 to detachably fix the iron cover 1350, and a fixing panel 1180 that extends from the other end of the partition panel 1160 to fix the cable 1400.

The mounting panel 1170 may be formed in a shape corresponding to that of a surface of the iron cover 1350, and may accommodate at least a portion of the iron cover 1350 therein.

The fixing panel 1180 may further include a hook or a ring that fixes a distal end of the handle 1311 or the cable 1400.

The cable 1400 may be accommodated inside the support portion 1120 at a location below the partition panel 1160. The cable 1400 accommodated in the support portion 1120 may be pressed against the partition panel 1160 to reduce a volume thereof.

The fixing panel 1180 may be rotatable below the partition panel 1160.

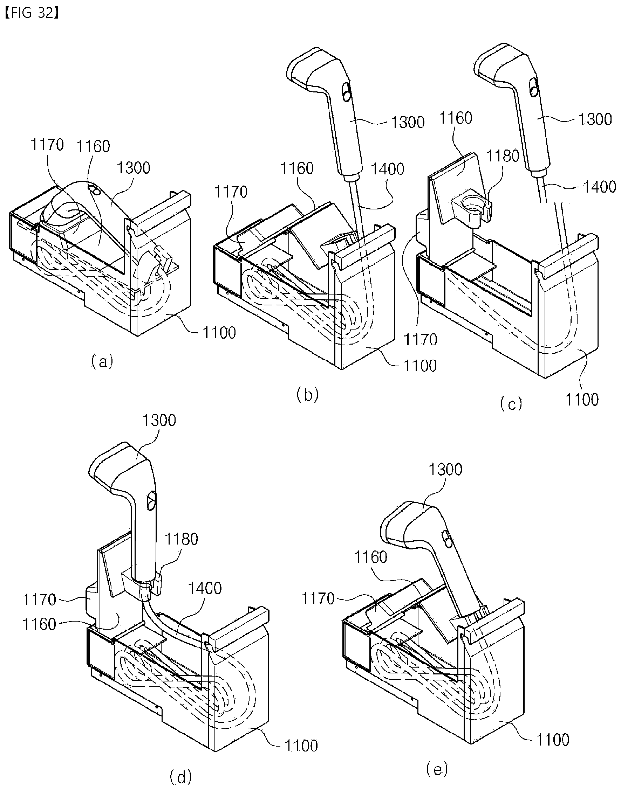

FIG. 32 shows an insertion/withdrawal scheme of the iron.

Referring to (a) in FIG. 32, the steam iron 1300 may be fixed to the mounting panel 1170, and the cable 1400 may be coupled to the fixing panel 1180. The partition panel 1160 may be in a state of pressurizing the cable 1400.

The cable 1400 may be accommodated inside the support portion 1120.

Referring to (b) in FIG. 32, when the withdrawal portion 1100 is in a state of being withdrawn forward from the housing 1200, the steam iron 1300 may be lifted upward. In this regard, the steam iron 1300 may be separated from the mounting panel 1170, and the fixing panel 1180 may be lifted upward as the cable 1400 is ifted upward. In this process, the partition panel 1160 and the mounting panel 1170 may be rotated upward in the support portion 1120.

Therefore, the partition panel 1160 may open the support portion 1120, so that the cable 1400 may be withdrawn.

Referring to (c) in FIG. 32, the partition panel 1160 and the mounting panel 1170 may be rotated to completely open the support portion 1120. The fixing panel 1180 may be rotated downward on the partition panel 1160 by a weight thereof.

With such process, the steam iron 1300 may be completely withdrawn from the support portion 1120 by the length of the cable 1400.

Referring to (d) in FIG. 32, the steam iron 1300 may be mounted on the fixing panel 1180. Further, the cable 1400 may be fully accommodated in the support portion 1120.

Referring to (e) in FIG. 32, the user may tilt the steam iron 1300 to be mounted on the mounting panel 1170. In this regard, the fixing panel 1180 may be rotated upward away from the partition panel 1160.

The mounting panel 1170 and the partition panel 1160 may be rotated to the support portion 1120. The partition panel 1160 may pressurize the cable 1400. With such process, the steam iron 1300 may be reinserted into the withdrawal portion 1100.

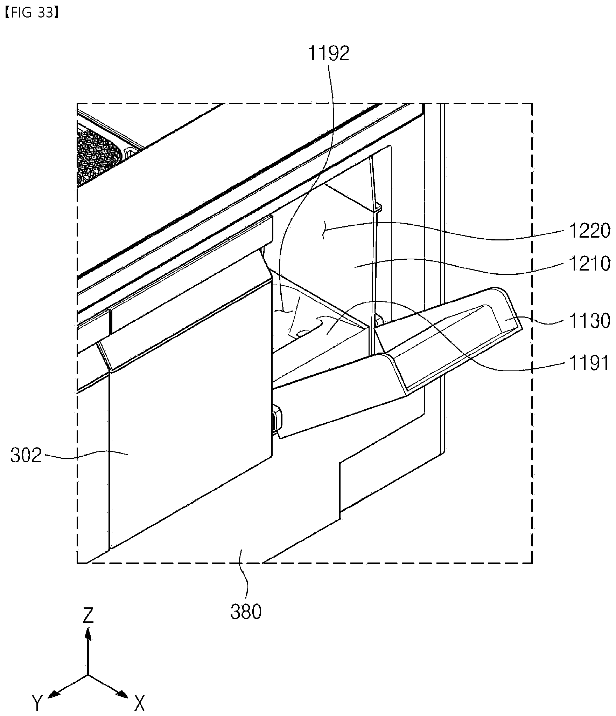

FIG. 33 shows another embodiment of the withdrawal portion.

In the iron module S of the present disclosure, the withdrawal portion 1100 may store the steam iron 1300 inside the housing body 1210, while selectively exposing the steam iron 1300 to the outside forwardly of the open surface 1220.

The iron module S may further include the cover 1130 of the withdrawal portion 1100 that is pivotable forward or rearward at a lower portion of the housing body 1210 to selectively expose the steam iron 1300.

The cover 1130 may be rotatably coupled atin front of the housing body 1210 to selectively expose the open surface 1220.

The cover 1130 may expose the steam iron 1300 that is accommodated in the housing body 1210, via simply pivoting. As a result, a component such as the drawer or the like for withdrawing the steam iron 1300 forward from the housing 1200 may be omitted.

The cover 1130 may have an area size that may block the open surface 1220 or the through-hole through which the iron module S passes in the detachment portion 380 from being exposed forward of the machine room 300. When the cover 1130 shields the open surface 1220 or the through-hole, the cover 1130 may be disposed parallel to the water supply tank 301.

The storage module 1000 may further include an iron seating portion 1190 that supports the iron cover 1350 of the steam iron 1300 in the lower portion of the housing 1200. The iron seating portion 1190 may accommodate at least a portion of the iron cover 1350 therein, and may be formed in a shape corresponding to the shape of the iron cover 1350.

The iron seating portion 1190 may include a seating body 1191 mounted in the housing body 1210, and a mounting groove 1192 depressed in a top surface of the mounting body 1191 to allow the iron cover 1350 to be mounted therein.

The iron seating portion 1190 may be accommodated in the housing body 1210 without being withdrawn to the open surface 1220.

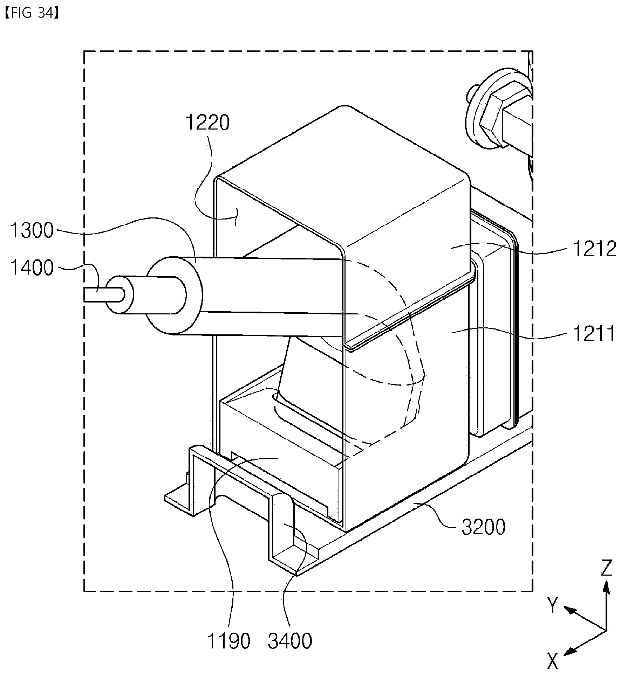

FIG. 34 shows a steam iron accommodated in the housing body.

The housing body 1210 may include a first body 1211 mounted on the base panel 3200, and a second body 1212 coupled to an upper portion of the first body 1211 to provide a space for storing the steam iron 1300 therein.

A front portion of the first body 1211 may form a lower portion of the open surface 1220, and a front portion of the second body 1212 may form an upper portion of the open surface 1220.

The iron seating portion 1190 that supports the steam iron 1300 may be installed inside the housing body 1210.

The base module 3000 may further include a pivoting stand 3400 disposed in front of the base panel 3200 and to which the cover 1130 is rotatably coupled.

The pivoting stand 3400 may be disposed in front of the iron seating portion 1190 and the open surface 1220.

The pivoting stand 3400 may be provided to rise to a vertical level at which a lower end of the cover 1130 is able to be disposed under the through-hole 385. Accordingly, the cover 1130 may selectively shield or open the through-hole 385.

The cover 1130 may open the through-hole 385 to expose the open surface 1220 and expose the steam iron 1300.

The cover 1130 may close the through-hole 385 to block the open surface 1220 and the steam iron 1300 from being exposed to the outside.

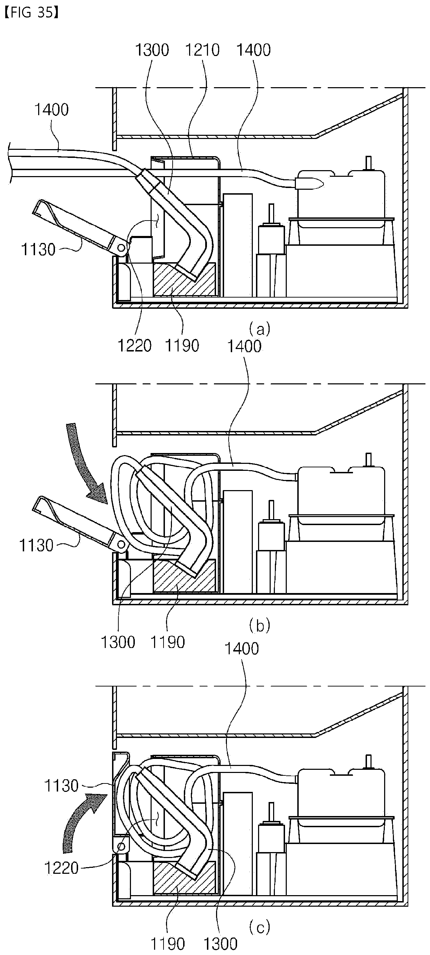

FIG. 35 shows a scheme of inserting and withdrawing the steam iron.

Referring to (a) in FIG. 35, the cover 1130 may pivot forward to open the through-hole 385. Accordingly, the steam iron 1300 may be withdrawn forward from the housing 1200 via the open surface 1220 and the through-hole 385.

In addition, the steam iron 1300 may be withdrawn and spray steam onto the surface of the laundry, and then be inserted into the through-hole 385 again and inserted into the open surface 1220 to be mounted on the iron seating portion 1190 starting with the head 1310.

Referring to (b) in FIG. 35, the user may roll up the cable 1400 and insert the cable 1400 into the through-hole 385. In this regard, a portion of the cable 1400 may be inserted into the housing body 1210, but a remaining portion thereof may be disposed between the open surface 1220 and the through-hole 385.

Referring to (c) in FIG. 35, the cover 1130 may close the through-hole 385. The iron cover 1350 of the steam iron 1300 may be seated on the iron seating portion 1190, and the handle 1311 may be supported on a rear surface of the cover 1130.

In addition, at least a portion of the cable 1400 may also be supported on the rear surface of the cover 1130.

Therefore, the user may easily insert the steam iron 1300 and the cable 1400 into the through-hole 385. Further, it is sufficient for the housing body 1210 to provide a space that accommodates only a portion of the steam iron 1300 and a portion of the cable 1400 as long as it accommodates the iron cover 1350 therein.

In other words, the space between the open surface 1220 and the through-hole 385 may be utilized as a space that accommodates the steam iron 1300 and the cable 1400 therein.

Therefore, overall length and volume of the iron module S may be reduced.

The present disclosure may be modified and implemented in various forms, so that the scope of rights thereof is not limited to the above-described embodiments. Therefore, when the modified embodiment includes elements of the claims of the present disclosure, it should be considered to fall within the scope of the present disclosure.

## Claims

1. A clothing treatment apparatus comprising:
a cabinet;
a door rotatably coupled to the cabinet;
an inner casing disposed inside the cabinet to provide an accommodating space where laundry is hung therein;
a machine room comprising a refreshing module including a base disposed below the inner casing, a circulation duct mounted or extended on the base to provide a flow channel where air inside the inner casing circulates, a heat supply configured to heat air flowing along the circulation duct, and a steam supply mounted on the circulation duct and configured to supply steam into the inner casing and an iron module including a steam iron disposed on a left side or a right side of the refreshing module and configured to supply steam to a surface of the laundry,
wherein the steam iron is withdrawn forward of the iron module.

2. The clothing treatment apparatus of claim 1, wherein the iron module includes a housing body disposed on a left side or a right side of the circulation duct to accommodate the steam iron therein, and an open surface defined in a front surface of the housing body and allowing the steam iron to be withdrawn forward of a machine room therethrough.

3. The clothing treatment apparatus of claim 2, wherein the open surface is exposable to the outside based on that the door is opened.

4. The clothing treatment apparatus of claim 2, wherein the iron module further includes an withdrawal portion accommodated in the housing body to withdraw the steam iron, and a cover disposed in front of the withdrawal portion to close the open surface.

5. The clothing treatment apparatus of claim 4, wherein the withdrawal portion is provided to expose the steam iron to the outside of the open surface by being withdrawn from the housing body via the open surface or coupled to be rotatably in front of the housing body.

6. The clothing treatment apparatus of claim 1, wherein the door includes a sealing member disposed on an inner surface thereof and in contact with a front surface of the inner casing.

7. The clothing treatment apparatus of claim 6, wherein the sealing member includes a partition member disposed between the inner casing and a machine room.

8. The clothing treatment apparatus of claim 6, wherein the door further includes a protruding surface protruding from the inner surface thereof and at least partially inserted into the accommodating space.

9. The clothing treatment apparatus of claim 8, wherein the protruding surface is disposed above the sealing member.

10. The clothing treatment apparatus of claim 9, wherein the protruding surface is provided to protrude from the inner surface of the door,
wherein the at least a portion protruding surface is provided to face a bottom surface of the inner casing in case that the door is closed.

11. The clothing treatment apparatus of claim 9, wherein a width of the protruding surface is set to be equal to or smaller than a width of the accommodating space.

12. The clothing treatment apparatus of claim 1, wherein the steam iron is set to operated and the refreshing module is set to be prevented from operating in case that the door is opened.

13. The clothing treatment apparatus of claim 1, wherein the steam iron is set to stop operating or be blocked from operating and the refreshing module is set to be operable in case that the door is closed.

14. The clothing treatment apparatus of claim 1, wherein the iron module further includes a heating module disposed on a left side or a right side of the circulation duct to generate steam to be supplied to the steam iron,
wherein the heating module is disposed behind the steam iron.

15. The clothing treatment apparatus of claim 12, wherein the steam iron includes:
a cable extending from the heating module and allowing heat and steam to flow therethrough; and
a handle coupled to a distal end of the cable and withdrawn from the housing forward of the machine room to supply heat and steam to the surface of the laundry.

16. A clothing treatment apparatus comprising:
a cabinet;
an inner casing disposed inside the cabinet to provide a space where laundry is hung therein;
a machine room positioned below the inner casing and provided to supply at least one of air and steam to the inside of the inner casing; and
an iron module mounted in the machine room and comprising a steam iron configured to supply at least one of heat and steam to the laundry installed therein,
wherein the steam iron is disposed to be exposable forward of the machine room.

17. The clothing treatment apparatus of claim 16, further comprising a door rotatably coupled to a front portion of the cabinet to open at least one of the inner casing and the machine room,
wherein the steam iron is disposed to be exposable forward of the machine room in case that the door is opened.

18. The clothing treatment apparatus of claim 17, wherein the steam iron is stored inside the machine room in case that the door is closed.

19. The clothing treatment apparatus of claim 16, wherein the machine room includes a circulation duct circulating air inside the inner casing, and a heat supply portion configured to heat air flowing along the circulation duct,
wherein the iron module further includes a housing disposed outside the circulation duct and accommodating the steam iron therein,
wherein the steam iron is disposed to be exposable forward of the machine room from the housing.

20. The clothing treatment apparatus of claim 19, wherein an entirety of the housing is disposed below a bottom surface of the inner casing.

21. A clothing treatment apparatus comprising:
a cabinet;
an inner casing disposed inside the cabinet to provide a space where laundry is hung therein;
a machine room disposed below the inner casing and provided to supply at least one of air and steam to the inside of the inner casing; and
an iron module mounted in the machine room and comprising a steam iron configured to supply at least one of heat and steam to the laundry installed therein,
wherein the machine room includes a circulation duct circulating air inside the inner casing, and a heat supply portion configured to heat air flowing along the circulation duct,
wherein the iron module includes:
a housing body disposed on a left side or a right side of the circulation duct to accommodate the steam iron therein; and
an open surface defined in a front surface of the housing body and exposing the steam iron forward of a machine room.

22. The clothing treatment apparatus of claim 21, wherein the housing body is provided to accommodate the steam iron therein so as to block the steam iron from being withdrawn via a top surface and both side surfaces thereof.

23. A clothing treatment apparatus comprising:
a cabinet;
an inner casing disposed inside the cabinet to provide a space where laundry is hung therein;
a machine room disposed below the inner casing and provided to supply at least one of air and steam to the inside of the inner casing; and
an iron module mounted in the machine room and comprising a steam iron configured to supply at least one of heat and steam to the laundry installed therein,
wherein the machine room further includes a circulation duct circulating air inside the inner casing, a heat supply portion configured to heat air flowing along the circulation duct, a steam supply portion configured to supply steam into the inner casing, a water supply tank disposed in front of the circulation duct to store water to be supplied to the steam supply therein, and a drainage tank collecting water condensed in the circulation duct and disposed on an opposite side of the water supply tank,
wherein the water supply tank and the drainage tank are both disposed in front of the circulation duct,
wherein the iron module is disposed on a left side or a right side of the circulation duct.

24. The clothing treatment apparatus of claim 23, wherein the water supply tank and the drainage tank are disposed adjacent to each other,
wherein the iron module is disposed to be spaced apart from one of the water supply tank and the drainage tank.

25. The clothing treatment apparatus of claim 23, wherein the iron module is disposed closer to the water supply tank than to the drainage tank.

26. The clothing treatment apparatus of claim 25, wherein the iron module further includes a heating module configured to receive water from the water supply tank and generate steam to be supplied to the steam iron,
wherein the heating module is disposed closer to the water supply tank than to the drainage tank.

27. A clothing treatment apparatus comprising:
a cabinet;
an inner casing disposed inside the cabinet to provide an accommodating space where laundry is hung therein;
a refreshing module including a base disposed below the inner casing, a circulation duct mounted or extended on the base to provide a flow channel where air inside the inner casing circulates, a heat supply portion configured to heat air flowing along the circulation duct, and a steam supply portion mounted on the circulation duct and configured to supply steam into the inner casing; and
an iron module including a steam iron disposed on a left side or a right side of the refreshing module and configured to supply steam to a surface of the laundry,
wherein the refreshing module further includes a main controller configured to control the heat supply portion and the steam supply portion,
wherein the iron module further includes an independent controller disposed independently of the main controller.
